# EUROPEAN PATENT SPECIFICATION

(11) **EP 4 203 856 B1**
(45) Date of publication and mention of the grant of the patent: **15.07.2026**
(21) Application number: 21787257.1
(22) Date of filing: 15.09.2021
(51) Int. Cl.: A61F 2/24, A61F 2/95

(54) **DELIVERY SYSTEMS FOR CARDIAC VALVE DEVICES**
FREISETZUNGSSYSTEME FÜR HERZKLAPPENVORRICHTUNGEN
SYSTÈMES DE POSE POUR DISPOSITIFS DE VALVULE CARDIAQUE

(30) Priority: 17.09.2020 US 202017024667; 05.03.2021 US 202117194113
(43) Date of publication of application: 05.07.2023
(73) Proprietor: Medtronic, Inc., Minneapolis, MN 55432 (US)
(72) Inventor: ZIMMERMAN, Neil, Menlo Park, California 94025 (US); MARTIN, Jeffrey, Menlo Park, California 94025 (US); DUERI, Jean-Pierre, Menlo Park, California 94025 (US); THAI, Erik, Menlo Park, California 94025 (US); JOHNSTON, Andrew, Menlo Park, California 94025 (US); SUTTON, Douglas, Menlo Park, California 94025 (US); ORTH, Cassandra, Menlo Park, California 94025 (US); O'GRADY, Robert, Menlo Park, California 94025 (US); GONZALEZ, Jose, Menlo Park, California 94025 (US); MCLEAN, Matthew, Menlo Park, California 94025 (US); KRISHNAMURTHY, Gaurav, Menlo Park, California 94025 (US)
(74) Representative: Zimmermann & Partner Patentanwälte mbB
(86) International application number: PCT/US2021/050538
(87) International publication number: WO 2022/060894

(56) References cited:
- EP-A1- 2 702 965
- WO-A1-2012/009006
- WO-A1-2017/035381

## Description

### CROSS-REFERENCE TO RELATED APPLICATIONS

This application claims priority to and the benefit of U.S. Patent Application No. 17/194,113, filed March 5, 2021, and titled "DELIVERY SYSTEMS FOR CARDIAC VALVE DEVICES, AND ASSOCIATED METHODS OF OPERATION," and U.S. Patent Application No. 17/024,667, filed September 17, 2020, and titled "DELIVERY SYSTEMS FOR CARDIAC VALVE DEVICES, AND ASSOCIATED METHODS OF OPERATION.

### TECHNICAL FIELD

The present technology generally relates to delivery systems for implanting cardiac valve devices via a minimally invasive procedure, such as an endovascular approach.

### BACKGROUND

Proper functioning of the mitral valve can be affected by mitral valve regurgitation, mitral valve prolapse, and/or mitral valve stenosis. Mitral valve regurgitation can occur when the leaflets of the mitral valve fail to coapt into apposition at peak contraction pressures such that blood leaks from the left ventricle into the left atrium. Several structural factors may affect the proper closure of the mitral valve leaflets. For example, an enlarged mitral annulus caused by dilation of heart muscle may prevent proper coaptation of the leaflets during systole. Other conditions involve a stretch or tear in the chordae tendineae-the tendons connecting the papillary muscles to the inferior side of the mitral valve leaflets-which may also affect proper closure of the mitral annulus. A ruptured chordae tendineae, for example, may cause a valve leaflet to prolapse into the left atrium due to inadequate tension on the leaflet. Abnormal backflow can also occur when the papillary muscles are compromised (e.g., due to ischemia) such that the affected papillary muscles do not contract sufficiently to effect proper closure during systole.

Mitral valve prolapse can occur when the mitral leaflets abnormally bulge up in to the left atrium, which can also lead to mitral valve regurgitation. Normal functioning of the mitral valve may also be affected by mitral valve stenosis, or a narrowing of the mitral valve orifice, which impedes of filling of the left ventricle during diastole.

Mitral valve regurgitation is often treated using diuretics and/or vasodilators to reduce the amount of blood flowing back into the left atrium. Other treatment methods, such as surgical approaches (open and intravascular), have also been used to either repair or replace the native mitral valve. For example, cinching or resecting portions of the dilated annulus are typical repair approaches. Cinching of the annulus has been accomplished by implanting annular or peri-annular rings which are generally secured to the annulus or surrounding tissue. Other repair procedures have also involved suturing or clipping of the valve leaflets into partial apposition with one another. Alternatively, more invasive procedures replace the entire valve with mechanical valves or biological tissue. These invasive procedures are conventionally done through large open thoracotomies and are thus very painful, have significant morbidity, and require long recovery periods.

However, with many repair and replacement procedures, the durability of the devices or improper sizing of annuloplasty rings or replacement valves may cause complications. Moreover, many of the repair procedures depend upon the skill of the cardiac surgeon since poorly or inaccurately placed sutures may affect the success of procedures.

Compared to other cardiac valves, the mitral valve presents unique challenges because portions of the mitral valve annulus have limited radial support from surrounding tissue and the mitral valve has an irregular, unpredictable shape. For example, the anterior wall of the mitral valve is bound by only a thin wall separating the mitral valve annulus from the inferior portion of the aortic outflow tract. As a result, significant radial forces on the mitral valve annulus are not acceptable as they could lead to collapse of the inferior portion of the aortic tract with potentially fatal consequences. Another challenge of the mitral valve anatomy is that the maze of chordae tendineae in the left ventricle makes navigating and positioning a deployment catheter much more difficult compared to other heart valves. Given the difficulties associated with current procedures, there remains the need for simple, effective, and less invasive devices and methods for treating dysfunctional heart valves. Additionally, since it is also difficult to deliver devices to the mitral valve, there also remains the need for effective and less invasive delivery systems to deliver the implantable cardiac devices to the mitral valve. WO 2017/035381 A1 relates to a delivery device and methods of transapical delivery of a replacement mitral valve.

### BRIEF DESCRIPTION OF THE DRAWINGS

Many aspects of the present disclosure can be better understood with reference to the following drawings. The components in the drawings are not necessarily to scale. Instead, emphasis is placed on clearly illustrating the principles of the present disclosure.
Figure 1 is a diagram of a mitral valve that may be accessed by a delivery system in accordance with embodiments of the present technology.
Figures 2A and 2B are a top view and a side view, respectively, of an implantable device that may be delivered to a heart of a subject (e.g., a patient) using a delivery system in accordance with embodiments of the present technology.
Figure 3 is a perspective side view of a delivery system configured in accordance with embodiments of the present technology.
Figure 4 is an enlarged isometric view of a distal portion of the delivery system of Figure 3 and the implantable device of Figures 2A and 2B in a partially-deployed position in accordance with embodiments of the present technology.
Figures 5A-5C are enlarged side views of a hub attachment mechanism of the delivery system of Figure 3 in a first position, a second position, and a third position, respectively, in accordance with embodiments of the present technology.
Figures 5D-5F are cross-sectional side views of the hub attachment mechanism of Figures 5A-5C in the first position, the second position, and the third position, respectively, in accordance with embodiments of the present technology.
Figures 6A-6C are an isometric view, a partial cross-sectional proximally-facing isometric view, and a partially cross-sectional distally-facing isometric view, respectively, of a hub shaft handle of the delivery system of Figure 3 in accordance with embodiments of the present technology.
Figures 7A and 7B are an isometric side view and an enlarged partial cross-sectional side view, respectively, of a distal plug on a core shaft of the delivery system of Figure 3 in accordance with embodiments of the present technology.
Figures 8A-8D are a distally-facing isometric view, a partially transparent side view, a partially transparent enlarged top view, and a partially transparent proximally-facing isometric view, respectively, of a core shaft handle of the delivery system of Figure 3 in accordance with embodiments of the present technology.
Figures 8E-8H are a partially transparent side view, a proximally-facing front view, a distally-facing isometric view, and another distally-facing isometric view, respectively, of a clip mount assembly and a cinch mount assembly of the core shaft handle of Figures 8A-8D configured in accordance with embodiments of the present technology.
Figure 9 is a flow diagram of a process or method for operating a delivery system to place the implantable device within a patient in accordance with embodiments of the present technology.
Figures 10A-10I are side views illustrating the implantable device and a distal portion of the delivery system during various stages of the method of Figure 9 in accordance with embodiments of the present technology.
Figure 11 is a flow diagram illustrating a process or method for releasing an implantable device from a delivery system in accordance with embodiments of the present technology.
Figures 12A-12C are side views illustrating the implantable device and a distal portion of the delivery system during various stages of the method of Figure 11 in accordance with embodiments of the present technology.
Figure 13A is a side view of a distal portion of a delivery catheter of Figure 3 configured in accordance with embodiments of the present technology.
Figure 13B is an enlarged cross-sectional view of a portion of the delivery catheter of Figure 3 taken along the line 13B-13B in Figure 13A.
Figures 14A-14D are a distally-facing isometric view, a partially transparent side view, a partially transparent enlarged top view, and a partially transparent proximally-facing isometric view, respectively, of a core shaft handle configured in accordance with additional embodiments of the present technology.
Figure 15 is an enlarged side view of a hub assembly configured in accordance with additional embodiments of the present technology.
Figures 16A and 16B are an enlarged side view and a front isometric view, respectively, of a hub assembly configured in accordance with additional embodiments of the present technology.

### DETAILED DESCRIPTION

Aspects of the present disclosure are directed generally to delivery systems for implanting a medical device, such as a valve repair device or a prosthetic heart valve, in a heart of a subject (e.g., a human patient). In several of the embodiments described below, for example, a delivery system includes a delivery catheter that holds an implantable medical device in a compressed configuration, a hub shaft extending through the delivery catheter, and a core shaft extending through the hub shaft. The hub shaft includes a hub that releasably engages a first portion of the medical device, and the core shaft includes a plug that releasably engages a second portion of the medical device. When the medical device is unsheathed from the delivery catheter, the hub shaft and the core shaft are independently movable (e.g., translatable) relative to one another to axially elongate or axially compress the medical device. When the medical device is properly positioned in the heart, the hub can be actuated to release the first portion of the medical device, and the plug can be actuated to release the second portion of the medical device.

In some embodiments, the medical device is configured to be implanted at a mitral valve of the heart of the subject. In such embodiments, the medical device can be implanted at the mitral valve by at least partially unsheathing the medical device from the delivery catheter in the left atrium above the mitral valve. The medical device is then longitudinally compressed to improve ease of steering by moving one or both of the hub shaft and the core shaft relative to one another. Next, the guide catheter, the delivery catheter, the hub shaft, and/or the core shaft can be used to steer the medical device toward and across the mitral valve annulus such that a portion of the medical device extends into the left ventricle to capture a portion of one or more native leaflets of the mitral valve and anchor the medical device in the sub-annular space behind the leaflet. After determining that the medical device is positioned and functioning properly, the hub and the plug can be actuated to disengage the medical device, leaving the medical device anchored to tissue surrounding the mitral valve.

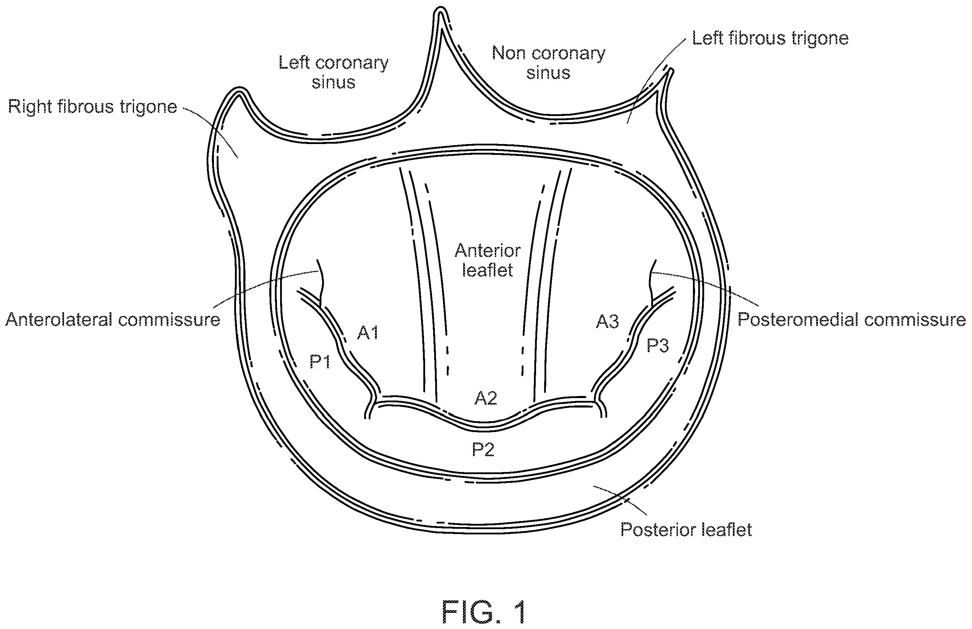

Specific details of several embodiments of the present technology are described herein with reference to Figures 1-12D. The present technology, however, can be practiced without some of these specific details. In some instances, well-known structures and techniques often associated with catheter-based delivery systems, prosthetic heart valves, etc., have not been shown in detail so as not to obscure the present technology. The terminology used in the description presented below is intended to be interpreted in its broadest reasonable manner, even though it is being used in conjunction with a detailed description of certain specific embodiments of the disclosure. Certain terms can even be emphasized below; however, any terminology intended to be interpreted in any restricted manner will be overtly and specifically defined as such in this Detailed Description section.

The accompanying Figures depict embodiments of the present technology and are not intended to be limiting of its scope. The sizes of various depicted elements are not necessarily drawn to scale, and these various elements can be arbitrarily enlarged to improve legibility. Component details can be abstracted in the Figures to exclude details such as position of components and certain precise connections between such components when such details are unnecessary for a complete understanding of how to make and use the present technology. Many of the details, dimensions, angles, and other features shown in the Figures are merely illustrative of particular embodiments of the disclosure. Accordingly, other embodiments can have other details, dimensions, angles, and features without departing from the scope of the present technology.

With regard to the terms "distal" and "proximal" within this description, unless otherwise specified, the terms can reference a relative position of the portions of a catheter subsystem with reference to an operator and/or a location in the vasculature. Also, as used herein, the designations "rearward," "forward," "upward," "downward," etc., are not meant to limit the referenced component to use in a specific orientation. It will be appreciated that such designations refer to the orientation of the referenced component as illustrated in the Figures; the systems of the present technology can be used in any orientation suitable to the user.

The headings provided herein are for convenience only and should not be construed as limiting the subject matter disclosed.

### I. Selected Embodiments of Implantable Devices and Associated Valve Anatomy

Figure 1 is a diagram of a mitral valve that may be accessed by a delivery system in accordance with embodiments of the present technology. The anterior leaflet has a semi-circular shape and attaches to approximately two-fifths of the annular circumference. The motion of the anterior leaflet defines an important boundary between the inflow (diastole) and outflow (systole) tracts of the left ventricle. The posterior leaflet of the mitral valve has a crescent shape and is attached to approximately three-fifths of the annular circumference. The posterior leaflet typically has two well-defined indentations which divide the leaflet into three individual scallops identified as P1 (lateral scallop), P2 (middle scallop), and P3 (medial scallop). The three corresponding segments of the anterior leaflet are identified as A1 (lateral segment), A2 (middle segment), and A3 (medial segment). The leaflet indentations aid in opening the posterior leaflet during diastole.

As shown in Figure 1, the mitral valve has anterolateral and posteromedial commissures which define a distinct area where the anterior and posterior leaflets come together at their insertion into the annulus. Sometimes the commissures exist as well-defined leaflet segments, but often this area is a subtle structure that can be identified using the following two anatomic landmarks: (a) the axis of corresponding papillary muscles, and (b) the commissural chordae, which have a specific fan-like configuration. Several millimeters of valvular tissue separate the free edge of the commissures from the annulus.

The mitral valve is an atrio-ventricular valve separating the left atrium from the left ventricle. The mitral annulus constitutes the anatomical junction between the left ventricle and the left atrium. The fixed ends of the leaflets are attached to the annulus. The anterior portion of the mitral annulus is attached to the fibrous trigones and is generally more developed than the posterior annulus. The right fibrous trigone is a dense junctional area between the mitral valve, tricuspid valve, non-coronary cusp of the aortic valve, and the membranous septum. The left fibrous trigone is situated at the junction of both left fibrous borders of the aortic valve and the mitral valve.

The mitral annulus is less well developed at the insertion site of the posterior leaflet. This segment is not attached to any fibrous structures, and the fibrous skeleton in this region is discontinuous. This posterior portion of the annulus is prone to increase its circumference when mitral regurgitation occurs in association with left atrial or left ventricular dilation. The mitral annulus is saddle-shaped, and during systole the commissural areas move proximally-that is, towards the roof of the atrium-while annular contraction also narrows the circumference. Both processes aid in achieving leaflet coaptation, which may be adversely affected by annular dilatation and calcification. The mitral annulus is surrounded by several important anatomic structures, including the aortic valve, the coronary sinus, and the circumflex artery. As a result, implanted cardiac devices at the mitral valve need to be positioned to accommodate the asymmetrical anatomy of the mitral valve without impacting the surrounding cardiac structures.

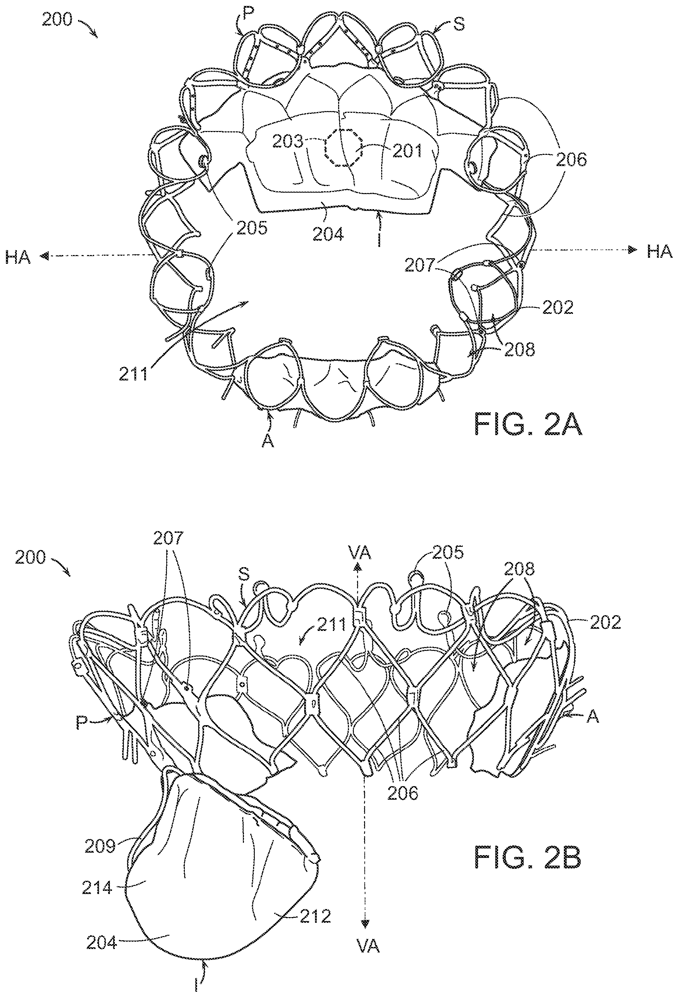

Figures 2A and 2B are a top view and a side view, respectively, of an implantable device 200 that may be delivered to a heart of a subject (e.g., a human patient) using a delivery system in accordance with embodiments of the present technology. Referring to Figures 2A and 2B together, in the illustrated embodiment the implantable device 200 is a valve repair device having an atrial-fixation member 202 (also referred to as an "anchoring member" or a "brim") and a coaptation member 204 (also referred to as a "baffle") extending from the atrial-fixation member 202 in a downstream direction. The atrial-fixation member 202 is configured to anchor the implantable device 200 to cardiac tissue proximate to a native mitral valve annulus and position the coaptation member 204 at a desired location with respect to the native valve anatomy of the heart. The coaptation member 204 is configured to displace at least a portion of one or more native leaflets of a cardiac valve and create a prosthetic coaptation surface for at least a portion of one or more of the other native leaflets of the cardiac valve. For example, when the implantable device 200 is deployed across the mitral valve annulus, the coaptation member 204 may extend in front of a central portion of the posterior leaflet (i.e., P2 of the posterior leaflet), pushing the posterior leaflet back toward the ventricular wall, such that the coaptation member 204 is positioned to coapt with the anterior leaflet during systole. The implantable device 200 is configured relative to a flow axis VA (Figure 2B) in the direction of blood flow from the atrium to the ventricle and a transverse axis HA (Figure 2A) at an angle (e.g., orthogonal) to the flow axis VA. The implantable device 200 has a posterior side portion P (e.g., a first side portion), an anterior side portion A (e.g., a second side portion), a superior end portion S (e.g., a first end portion), and an inferior end portion I (e.g., a second end portion).

In some embodiments, the implantable device 200 can include some features generally similar or identical to the implantable devices described in (i) U.S. Patent Application No. 16/044,447, titled "PROSTHETIC LEAFLET DEVICE," and filed July 24, 2018, (ii) International Patent Application No. PCT/US2018/061126, titled "LEAFLET EXTENSION FOR CARDIAC VALVE LEAFLET," and filed November 14, 2018, (iii) U.S. Patent Application No. 16/745,246, titled "IMPLANTABLE COAPTATION ASSIST DEVICES WITH SENSORS AND ASSOCIATED SYSTEMS AND METHODS," and filed January 16, 2020, and/or (iv) U.S. Patent Application No. 16/817,464, titled "CARDIAC VALVE REPAIR DEVICES WITH ANNULOPLASTY FEATURES AND ASSOCIATED SYSTEMS AND METHODS," and filed March 12, 2020. Any of several prosthetic valve repair or replacement devices could similarly be used with delivery systems in accordance with the present technology, including complete mitral valve replacement devices. And, in addition to mitral valve devices, other valve repair or replacement devices could be delivered to the tricuspid, aortic, and pulmonic valves using delivery systems in accordance with the present invention.

The atrial-fixation member 202 can be formed of a mesh, such as a braid or laser-cut stent-like structure, including a plurality of interconnected wires or struts 206 which together define a plurality of openings or cells 208 (e.g., diamond-shaped openings) arranged in one or more rows. The struts 206 can be configured to self-expand from a collapsed delivery state (not shown) to an expanded deployed state shown in Figures 2A and 2B. The struts 206 can be formed of any biocompatible material such as, for example, stainless steel, nickel-titanium alloys (e.g., nitinol), and/or other suitable stent materials. The atrial-fixation member 202 can have a generally circular, oval, or D-like shape in the deployed state and define an open central lumen 211 (also referred to as an "opening") that allows blood to pass therethrough along the flow axis VA. When the implantable device 200 is configured to repair a native mitral valve, the atrial-fixation member 202 can be shaped to conform to the walls of the left atrium just above the mitral annulus to secure the implantable device 200 to the supra-annular tissue. After a period of time post-implantation (e.g., 3 days, 2 weeks, 1 month, 2 months, the atrial-fixation member 202 or portions thereof become covered by a layer of tissue, and this tissue ingrowth adheres the implantable device 200 permanently to the atrial wall. In some embodiments, the atrial-fixation member 202 has a semi-circular or other shape that does not extend fully around the circumference of the native valve. In some embodiments, the atrial-fixation member 202 may also or alternatively include one or more portions that press against sub-annular tissue to provide sub-annular device fixation.

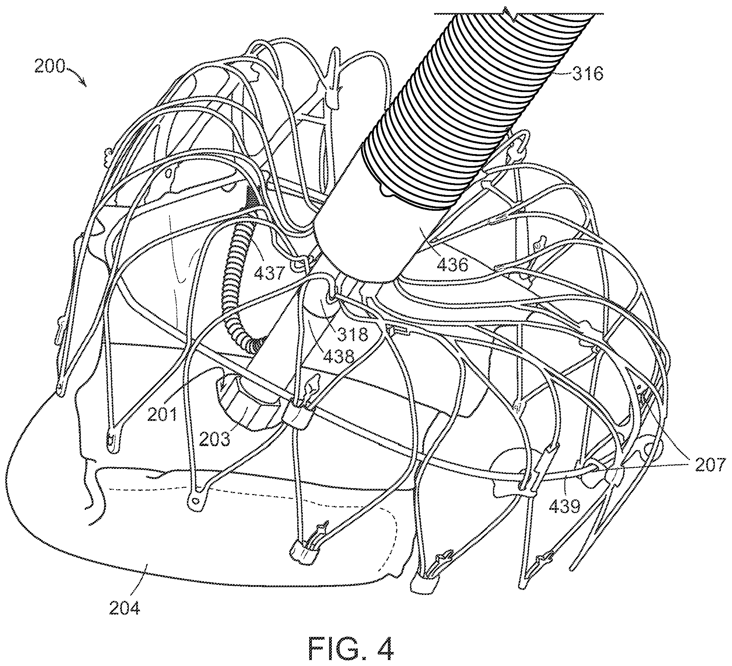

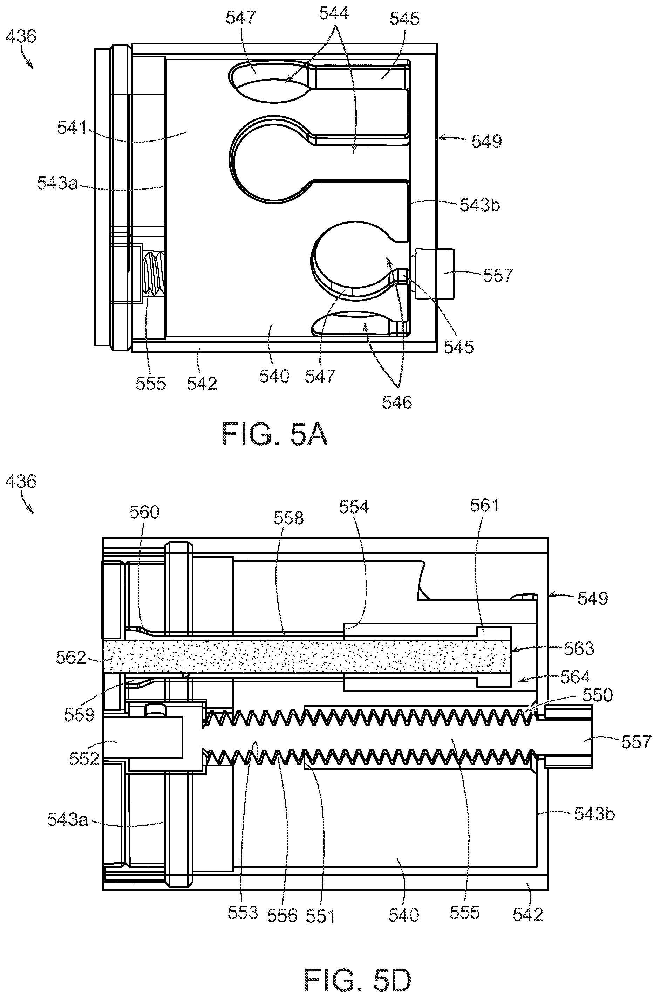

In some embodiments, the atrial-fixation member 202 can include connectors 205 that are configured (e.g., sized, shaped, and/or positioned) to engage with a mating feature on the delivery system, as described in detail below with reference to Figures 5A-5F. As shown in Figure 2B, for example, the connectors 205 may be extend from the struts 206 such that the connectors 205 are positioned near or at the superior end portion S of the implantable device 200. In some embodiments, the atrial-fixation member 202 includes one or more eyelets 207 configured to receive one or more tendons (e.g., a cinch tendon 439 illustrated in Figure 4) that aids in packing (e.g., compressing), delivering, orienting, and/or retrieving the implantable device 200. For example, the tendons can help facilitate cinching (e.g., radially compressing) of the atrial-fixation member 202. The eyelets 207 can be metal portions of the atrial-fixation member 202, or can be separate filaments/wires forming loops and attached to the atrial-fixation member 202.

As shown in Figures 2A and 2B, the coaptation structure 204 extends away from a downstream end portion of the atrial-fixation member 202 along the flow axis VA and at least a portion of the coaptation member 204 extends radially inward from the atrial-fixation member 202 into the central lumen 211 to approximate a closed position of a native leaflet. The coaptation member 204 can be substantially stationary (e.g., little to no movement) during cardiac cycles such that the position of the coaptation member 204 relative to the atrial-fixation member 202 is at least substantially fixed in the deployed state. Thus, unlike native leaflets that move back and forth to open and close the native valve, the coaptation member 204 remains stationary during diastole and systole.

The coaptation member 204 can have an anterior portion 212 (Figure 2B) with a smooth, atraumatic surface for coapting with at least a portion of one or more native leaflets and a posterior portion 214 (Figure 2B) configured to displace and, optionally, engage at least a portion of another native leaflet. The coaptation member 204 can be made from a plurality of struts that form a basket-like or frame-like structure (e.g., a mesh structure, a laser cut stent frame) with an at least partially hollow interior and a covering (e.g., a fabric) extending over at least a portion of the struts to provide a smooth suitable surface for coaptation at the anterior portion 212. The covering may also extend over the struts along the posterior portion 214 and between the anterior and posterior portions 212, 214 in a manner that forms lateral sidewalls. The baffle 204 or portions thereof can be integral with the atrial-fixation member 202 such that, for example, the coaptation member 204 is manufactured from the same frame including the struts 206. In other embodiments, the baffle 204 can be a separate structure that is connected to a portion of the atrial-fixation member 202 during manufacturing. In some embodiments, the baffle 204 can include a biocompatible foam which is attached to the structure of the baffle 204 and/or to the atrial-fixation member 202.

In the illustrated embodiment, the baffle 204 further includes a normally-closed clip 209 (obscured in Figure 2A) depending from its posterior surface which can be opened to extend behind the native leaflet the coaptation member 204 displaces. The clip 209 may grasp the native leaflet and/or engage sub-annular cardiac tissue for sub-annular stabilization of the implantable device 200. In some embodiments, for example, the clip 209 reaches under the central portion (i.e., P2) of the posterior leaflet up to the sub-annular space. A tendon (made of suture or nitinol wire) can actuate the clip 209 by way of a lever attached to the clip 209. The lever may be a nitinol wire or laser cut nitinol or Co-Cr sheet.

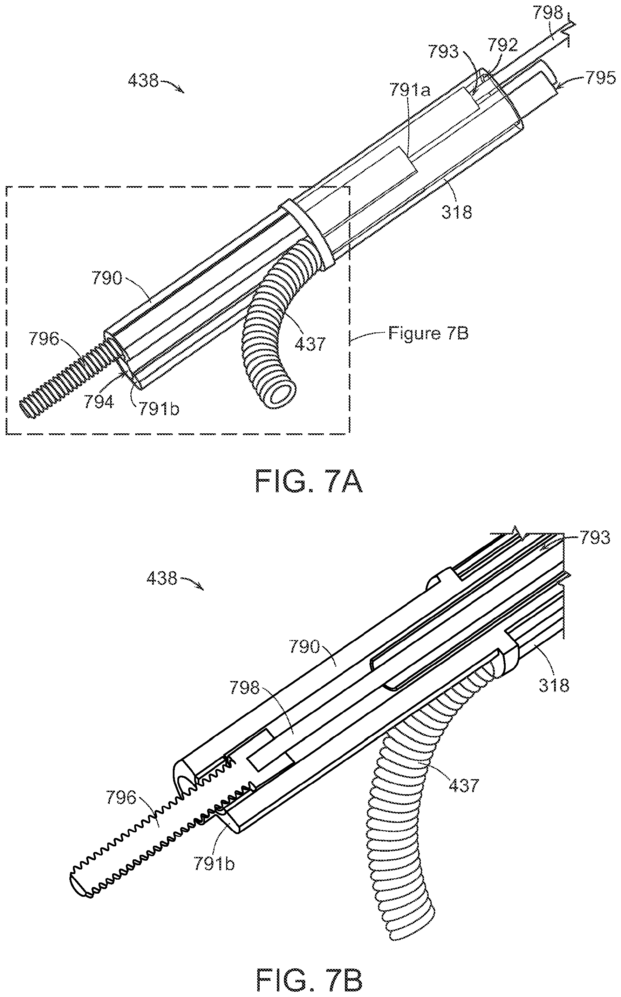

As shown in Figure 2A, the baffle 204 can further include a delivery attachment member 203 (shown in broken lines) positioned within the hollow interior of the baffle 204. The delivery attachment member 203 can be a threaded nut or other type of connector configured to mate with a corresponding portion (e.g., a screw) of the delivery system, as described in greater detail below with reference to Figures 4, 7A, and 7B. In some embodiments, the delivery attachment member 203 is accessible via a flap or opening 201 (Figure 2A) formed in the baffle 204 (e.g., in portion of the baffle facing the superior end portion S of the implantable device 200).

The implantable device 200 may be inserted via a femoral vein sheath to traverse the inferior vena cava to the right atrium. The implantable device 200 is then inserted into the left atrium via a puncture of the interatrial septum. In several applications, the implantable device 200 is delivered to a target location within the mitral valve to function properly. This means appropriate positioning along the flow axis VA, correct radial positioning relative to the central axis of the valve, correct rotational orientation to specific landmarks such as the middle (P2) portion of the native posterior leaflet, and correct angular positioning relative to the flow axis and the transverse axis. In some embodiments, the implantable device 200 may also be repositioned during the delivery process to, for example, correct for misalignment or inappropriate positioning. During deployment and release of the implantable device 200, the delivery system can retain the implantable device 200 in a stationary position at the desired location and in the desired orientation relative to the native valve. Furthermore, the delivery system may be configured to allow the implantable device 200 to be re-sheathed, repositioned, and/or removed before being released from the delivery system. Delivery systems of the present technology can achieve all the above-mentioned advantages in a user-friendly system. Additionally, several embodiments of delivery systems in accordance with the present technology have a small overall diameter, such as approximately 15 to 30 French.

### II. Selected Embodiments of Delivery Systems

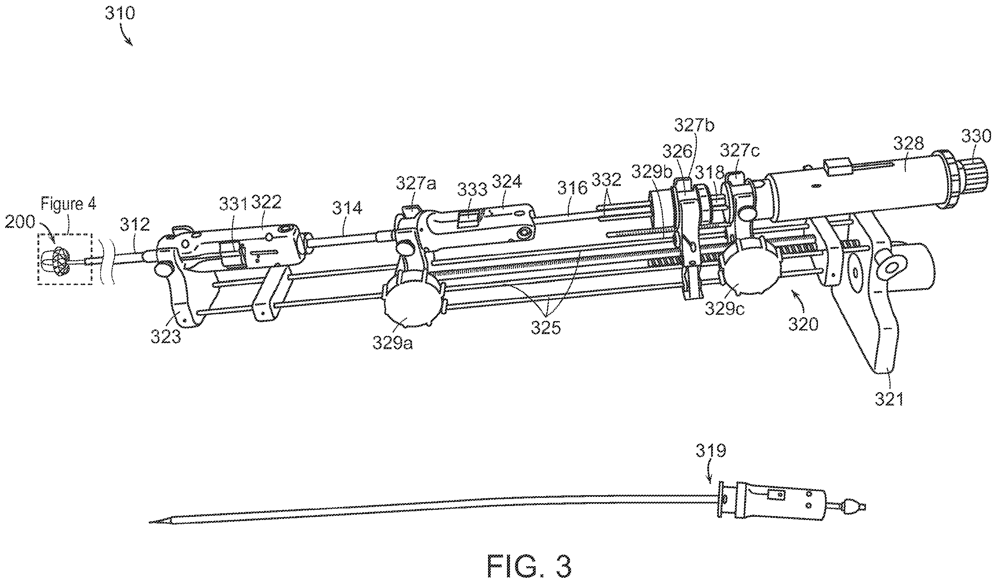

Figure 3 is a perspective side view of a delivery system 310 configured in accordance with embodiments of the present technology. The delivery system 310 can be used to deliver an implantable device, such as the implantable valve repair device 200 of Figures 2A and 2B, to a heart of a subject (e.g., a human patient). In the illustrated embodiment, the delivery system 310 includes four nested/coaxial catheter/shaft structures: (i) an outer guide catheter 312, (ii) a delivery catheter 314 (also referred to as a "sleeve") configured to extend at least partially through the guide catheter 312, (iii) a hub shaft 316 configured to extend at least partially through the delivery catheter 314, and (iv) a core shaft 318 configured to extend at least partially through the hub shaft 316 (collectively "catheters 312-318" or "shafts 312-318"). The catheters 312-318 can be individually manipulated and/or moved relative to one another to facilitate deployment of the implantable device 200. More specifically, in the illustrated embodiment (i) the guide catheter 312 is coupled to a guide catheter handle 322, (ii) the delivery catheter 314 is coupled to a delivery catheter handle 324, (iii) the hub shaft 316 is coupled to a hub shaft handle 326, and (iv) the core shaft 318 is coupled to a core shaft handle 328 (collectively "handles 322-328" or "handle assembly"). In some embodiments, the delivery system 310 further includes a dilator assembly 319 configured to be advanced/retracted through the guide catheter 312 prior to introduction of the delivery catheter 314, the hub shaft 316, and/or the core shaft 318.

In some embodiments, the handles 322-328 and/or portions of the catheters 312-318 are coupled/mounted to a common handle support assembly 320 (also referred to as a "rack assembly" or "control rack") that facilitates relative movement between the individual catheters 312-318, while maintaining the handles 322-328 in a stable, supported position and inhibiting unwanted movement therebetween. The support assembly 320 can include a proximal fixed portion 321 (e.g., a first stand), a distal fixed portion 323 (e.g., a second stand), and one or more tracks 325 extending at least partially between the proximal and distal fixed portions 321, 323. In the illustrated embodiment, the guide catheter handle 322 is removably mounted to the distal fixed portion 323.

As shown in Figure 3, the support assembly 320 can further include a plurality of mounts 327 (identified individually as first through third mounts 327a-327c, respectively) slidably coupled to one or more of the tracks 325. The first mount 327a is configured to receive and secure the delivery catheter handle 324, and includes a first actuation member 329a (e.g., a wheel, slider, knob, button) for individually moving the first mount 327a-and the delivery catheter handle 324 and the delivery catheter 314 coupled thereto-linearly along the tracks 325 relative to the other ones of the handles 322-328. The second mount 327b is positioned proximal of the first mount 327a and is configured to receive and secure the hub shaft handle 326. Similarly, the second mount 327b includes a second actuation member 329b for individually moving the second mount 327b and the hub shaft handle 326. Likewise, the third mount 327c is positioned proximal of the second mount 327b, is configured to receive and secure the core shaft handle 328, and includes a third actuation member 329c for individually moving the third mount 327c and the core shaft handle 328. In the illustrated embodiment, the support assembly 320 further includes a linear drive mechanism 330 (e.g., a screw drive, a rack and pinion) configured to jointly advance/retract the delivery catheter handle 324, the hub shaft handle 326, and the core shaft handle 328 without moving the handles relative to one another. In some embodiments, the linear drive mechanism 330 is coupled to only a subset of the handles 322-328 and/or the system 310 can include more than one linear drive mechanism to linearly advance one or more of the handles 322-328.

The guide catheter 312 and the delivery catheter 314 can each have varying stiffnesses along a length thereof and/or can be steerable catheters that allow the catheters 312, 314 to deflect along one or more axes. In some embodiments, for example, the guide catheter handle 322 includes a guide actuation member 331 (e.g., a wheel, lever, knob, slider) that is actuatable to deflect a distal portion of the guide catheter 312. More specifically, the guide actuation member 331 can be coupled to a pull wire that is attached to a pull ring fixed at a distal portion of the guide catheter 312. Similarly, the delivery catheter handle 324 can include a delivery actuation member 333 that is actuatable to deflect a distal portion of the delivery catheter 314. In some embodiments, the guide catheter 312 has a diameter of less than about 30 French (e.g., about 29.5 French or less) and the delivery catheter 314 has a diameter of about 26 French or less.

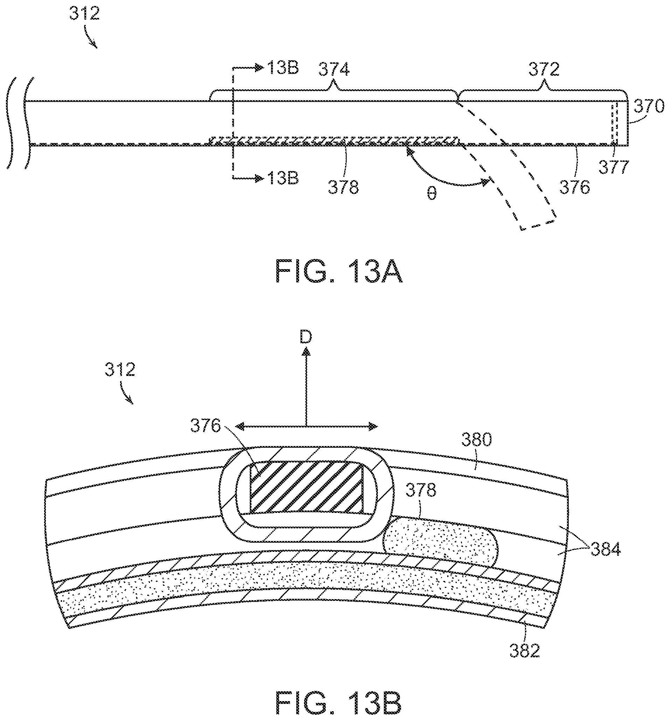

More specifically, Figure 13A is a side view of a distal portion of the delivery catheter 314 configured in accordance with embodiments of the present technology. In the illustrated embodiment, the delivery catheter 314 includes a distal terminus 370, a first region 372 (e.g., a distal region) adjacent the distal terminus 370, and a second region 374 (e.g., a proximal region) adjacent to and proximal of the first region 372. Figure 13B is an enlarged cross-sectional view of a portion of the delivery catheter 314 (e.g., a portion of a wall of the delivery catheter 314) taken along the line 13B-13B in Figure 13A. In the illustrated embodiment, the delivery catheter 314 includes an outer jacket layer 380, an inner liner layer 382, and one or more layers of braided and/or coiled reinforcement material 384 between the inner liner and outer jacket layers 382, 380 (collectively "layers 380-384"). The inner liner and outer jacket layers 382, 380 can be formed of polytetrafluoroethylene (PTFE), plastic, elastomer, thermoplastic elastomer (TPE) (e.g., a TPE manufactured by Arkema S.A., of Colombes, France, such as the TPEs manufactured under the trademark "Pebax"), nylon, and/or other suitable materials, and the layers of braided and/or coiled material 384 can be formed from metal wires and/or stiffer polymers.

Referring to Figures 13A and 13B together, the delivery catheter 314 can further include a pull wire 376 (obscured by the outer jacket layer 380 in Figure 13A and shown schematically) extending through a lumen between some of the layers 380-384 and having (i) a distal end or portion coupled to a pull ring 377 (obscured by the outer jacket layer 380 in Figure 13A and shown schematically) positioned in the first region 372 near the distal terminus 370 and (ii) a proximal end or portion coupled to the delivery actuation member 333 of the delivery catheter handle 324 (Figure 3). Actuation of the delivery actuation member 333 can pull the pull wire 376 to cause the first region 372 and/or the second region 374 to deflect (e.g., in the direction indicated by arrow D in Figure 13B).

In some embodiments, the delivery catheter 314 can further include a spine 378 (also referred to as an "elongate member") extending at least partially through/along one or more of the layers 380-384. In the illustrated embodiment, the spine 378 extends along the second region 374 but not the first region 372. In some embodiments, the first region 372 can have a length of between about 10-50 millimeters (e.g., about 20 millimeters), and the second region 374 and the spine 378 can have a length of between about 20-80 millimeters (e.g., about 50 millimeters). The spine 378 can be formed of metal or other suitably rigid materials and is positioned adjacent to (e.g., parallel to) the pull wire 376 along the second region 374. In some embodiments, the spine 378 is a metal wire welded (e.g., tack-welded) to the coil reinforcement wire 384 along the second region 374. In some embodiments, when the pull wire 376 is pulled to deflect the delivery catheter 314, the spine 378 inhibits or even prevents the second region 374 of the delivery catheter 376 from flexing in the direction indicated by the arrow D. That is, the spine 378 can inhibit the second region 374 from deflecting while still permitting the first region 372 to deflect in the direction of arrow D. Accordingly, as shown in phantom in Figure 13A, an angle θ between the first and second regions 372, 374 after actuation of the pull wire 376 can be greater than the angle θ would be without the spine 378. At the same time, the spine 378 can still permit the second region 374 to bend in directions other than the direction of arrow D, such as along an axis O orthogonal to the direction of arrow D. In some aspects of the present technology, this selective flexibility can facilitate advancement of the delivery catheter 314 through the guide catheter 312 (Figure 3) and/or permit the guide catheter 312 to bend the delivery catheter 314 along the axis O. That is, the spine 378 can provide the delivery catheter 314 with selective flexibility-allowing the delivery catheter 314 to bend along the axis O while remaining stiff and inhibiting bending in the direction of arrow D when the pull wire 376 is pulled.

During a delivery procedure using the delivery system 310, the distal portion of the delivery catheter 314 retains the implantable device 200 in the compressed delivery state and, upon reaching a target region (e.g., in the left atrium), begins to deploy (e.g., unsheathe) the implantable device 200 by retracting the delivery catheter 314 and/or advancing the implantable device 200 beyond the distal terminus of the delivery catheter 314. This allows the implantable device 200 to partially expand toward the deployed state, while still being releasably secured to a distal portion of the hub shaft 316 and a distal portion of the core shaft 318. In Figure 3, for example, the implantable device 200 is shown in a partially-deployed position (also referred to as a "partially-deployed state") in which the implantable device 200 (i) has been advanced out of the distal portion of the delivery catheter 314, but (ii) is still secured to the hub shaft 316 and the core shaft 318 to allow for controlled movement (e.g., linear, rotational) and further deployment of the partially-deployed implantable device 200.

More specifically, Figure 4 is an enlarged isometric view of a distal portion of the delivery system 310 and the implantable device 200 in the partially-deployed position in accordance with embodiments of the present technology. In the illustrated embodiment, the hub shaft 316 includes a distal hub assembly 436 (described in further detail below with reference to Figures 5A-5F) that releasably engages a portion (e.g., the connectors 205 (Figure 2)) of the implantable device 200. The core shaft 318 includes a distal plug assembly 438 (also referred to as a "core plug" or "plug,"; described in further detail below with reference to Figures 7A and 7B) extending through the opening 201 in the baffle 204 and that is releasably coupled to the delivery attachment member 203. Accordingly, relative linear translation between the hub shaft 316 and the core shaft 318 can elongate and/or shorten (e.g., axially, longitudinally, etc.) the implantable device 200 and, more particularly, the atrial-fixation member 202. In the illustrated embodiment, the plug assembly 438 further includes a flexible tube 437 (e.g., a coiled tube) extending therefrom to route a cinch tendon 439 (e.g., a suture) from the proximal portion of the delivery system 310 (e.g., near or at the handles 322-328) to the eyelets 207 of the atrial-fixation member 202.

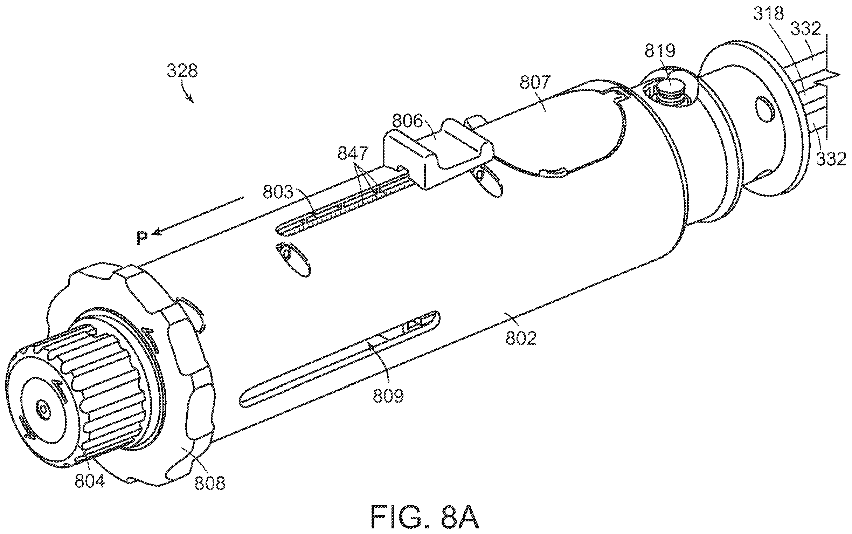

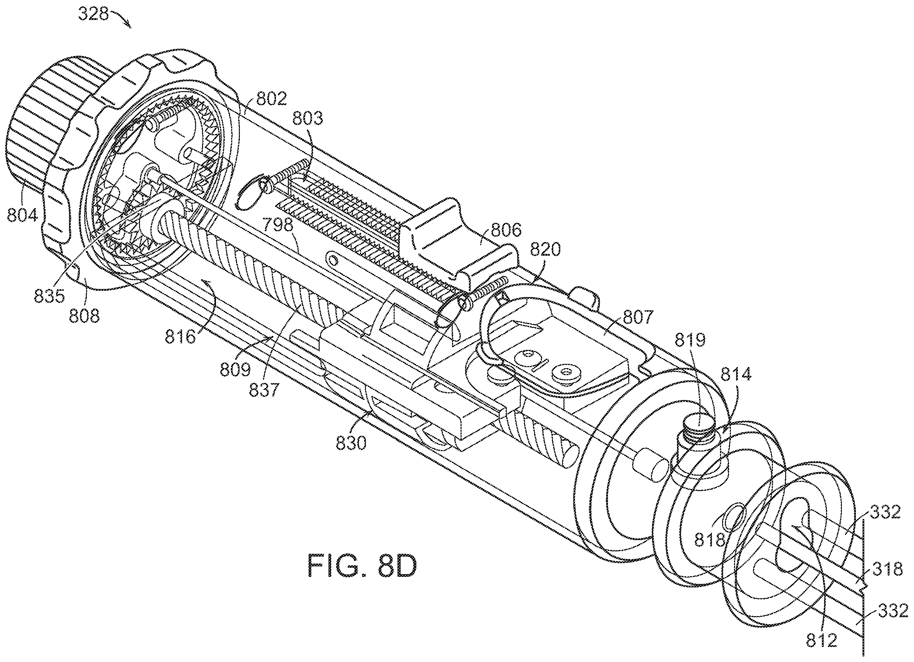

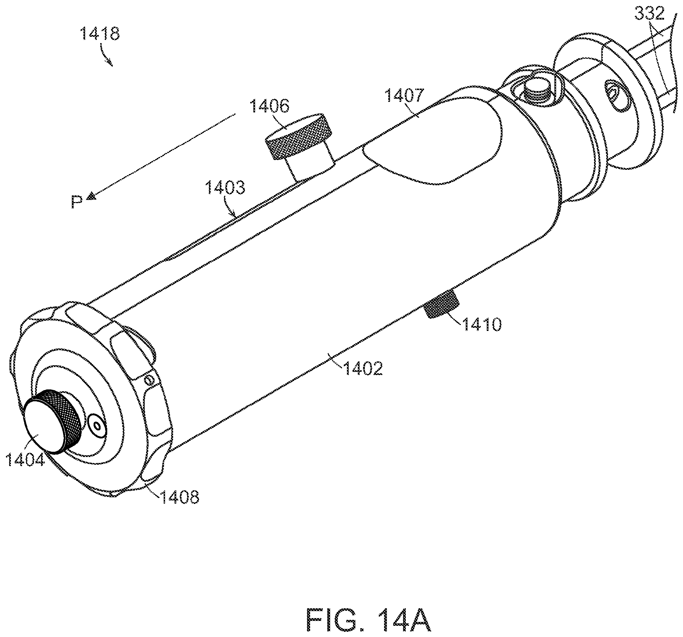

Referring to Figure 3 and 4 together, in some embodiments one or more of the handles 322-328 can be coupled together using means that inhibit relative rotational motion of the handles 322-328. In the illustrated embodiment, for example, the hub shaft handle 326 is slidably coupled to the core shaft handle 328 via a pair of rails 332 (e.g., rigid members, such as rods) that inhibit or even prevent the hub shaft handle 326 and the core shaft handle 328 from rotating relative to one another. Thus, the hub shaft 316 and the core shaft 318 extending from the corresponding hub shaft and core shaft handles 326 and 328 can be rotationally fixed relative to each other (or at least substantially so) during implant procedures. Accordingly, the rails 332 can inhibit or even prevent twisting of the implantable device 200, which has portions coupled to both the hub shaft 316 and the core shaft 318, during an implant procedure that could otherwise arise from rotation of the hub shaft 316 relative to the core shaft 318. In the illustrated embodiment, the hub shaft handle 326 is coupled to the core shaft handle 328 via two rails, whereas in further embodiments the two handles 326, 328 may be coupled together with a single rail, more than two rails (e.g., three, four, five, or more rails), and/or other coupling mechanisms that maintain the rotational alignment of the handles 326, 328 and the catheters and shafts coupled thereto. In other embodiments, the handles 326, 328 can be integrated into a single handle including the various components and functionality described in detail below with reference to Figures 8A-8H and/or 14A-14D. In some embodiments, other handles or subsets of handles 322-328 are coupled together in a similar manner to avoid relative motion between the handles 322-328.

Figures 5A-5C are enlarged side views of the hub assembly 436 of Figure 4 in a first position, a second position, and a third position, respectively, in accordance with embodiments of the present technology. Figures 5D-5F are side cross-sectional views of the hub assembly 436 in the first position, the second position, and the third position, respectively, in accordance with embodiments of the present technology. Referring first to Figures 5A and 5D together, the hub assembly 436 includes an inner hub component 540 and an outer hub component 542 (also referred to as a "capsule"). The outer hub component 542 is shown as partially transparent in Figures 5A-5F for the sake of clarity. The inner hub component 540 includes an outer surface 541, a proximal side or edge 543a, and a distal side or edge 543b. In the illustrated embodiment, the outer surface 541 of the inner hub component 540 includes a plurality/group of first recesses 544 and a plurality/group of second recesses 546 that extend from the distal edge 543b toward the proximal edge 543a. The first and second recesses 544, 546 are configured (e.g., shaped, sized, and/or positioned) to receive and secure corresponding ones of the connectors 205 of the implantable device 200 (Figures 2A and 2B). In some embodiments, the first and second recesses 544, 546 can have a generally similar shape including (i) a first portion 545 having a generally elongate (e.g., rectangular) shape for receiving a portion of a corresponding one of the struts 206 (Figures 2A and 2B) and (ii) a second portion 547 having a generally circular shape for receiving a corresponding one of the connectors 205 (Figures 2A and 2B). In other embodiments, the first and second recesses 544, 546 can have other suitable shapes for receiving and securing the connectors 205 and/or other features at the superior end portion S of the implantable device 200.

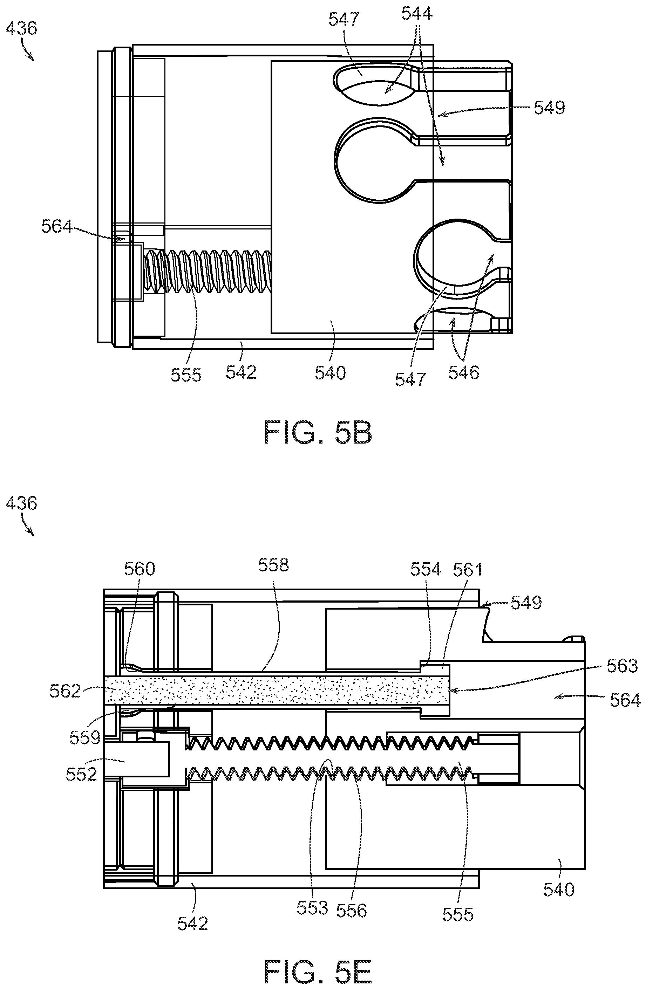

In the illustrated embodiment, the first recesses 544 are circumferentially spaced apart from the second recesses 546. That is, the first recesses 544 are positioned along/in a first circumferential portion of the outer surface 541, while the second recesses 546 are positioned along a second circumferential portion of the outer surface 541. In some embodiments, the first portions 545 of the second recesses 546 are shorter than the first portions 545 of the first recesses 544 (e.g., in a direction extending between the proximal and distal edges 543a, b). Accordingly, the second portions 547 of the second recesses 546 can be positioned closer to the distal edge 543b than the second portions 547 of the first recesses 544. In some embodiments, the first recesses 544 are configured to receive corresponding ones of the connectors 205 positioned at the anterior side portion A of the implantable device 200 (Figures 2A and 2B), while the second recesses 546 are configured to receive corresponding ones of the connectors 205 positioned at the posterior side portion P of the implantable device 200. As described in greater detail below with reference to Figures 5B, 5C, 5E, 5F and 11-12D, this arrangement can facilitate a two-stage release of the implantable device 200 in which the connectors 205 positioned at the posterior side portion P of the implantable device 200 are released from the hub assembly 436 before the connectors 205 positioned at the anterior side portion A of the implantable device 200. In some embodiments, the inner hub component 540 can include recesses that are aligned for a single-stage deployment, or recesses that are arranged around the outer surface 541 to facilitate deployment in three or more stages.

In the illustrated embodiment, the outer hub component 542 includes a distal opening 549 and, in the first position illustrated in Figures 5A and 5D, each of the first and second recesses 544, 546 are positioned proximal of the distal opening 549. Accordingly, in the first position, the connectors 205 of the implantable device 200 are secured/restrained in the first and second recesses 544, 546 by the outer hub component 542.

As best seen in Figure 5D, the inner hub component 540 includes/defines a drive lumen 550 and a lock lumen 564 both extending at least partially from the proximal edge 543a toward the distal edge 543b. The drive lumen 550 includes a stepped stop surface 551 such that the drive lumen 550 has a larger dimension (e.g., diameter) distal of the stop surface 551. The drive lumen 550 further includes a threaded portion 553 proximal of the stop surface 551. Likewise, the lock lumen 564 includes a stepped or tapered stop surface 554 such that the lock lumen 564 has a larger dimension distal of the stop surface 554.

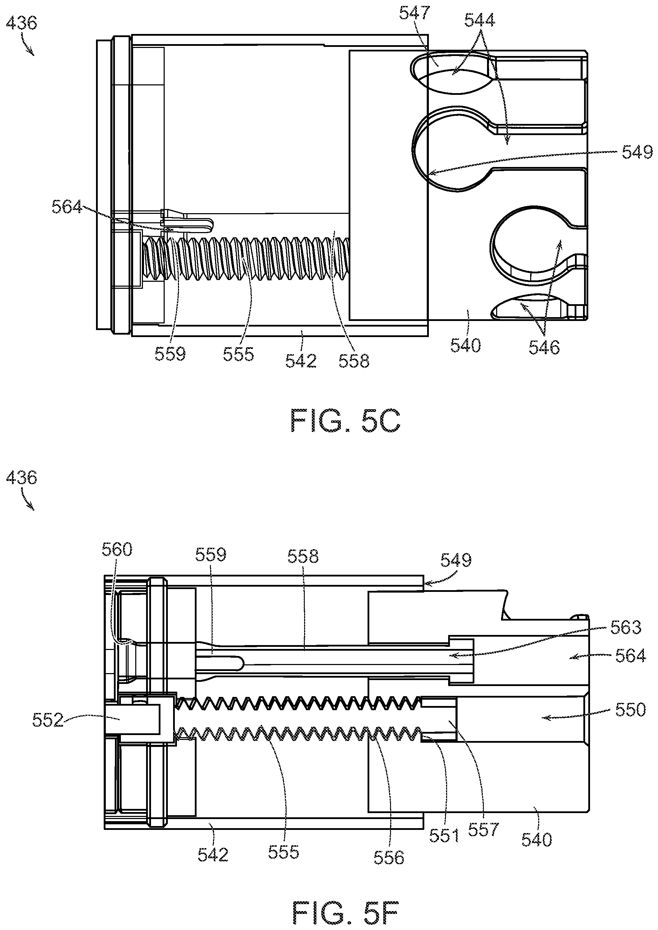

The hub assembly 436 can further include a lead screw 555 extending at least partially through the drive lumen 550 and operably coupling the inner hub component 540 to a drive shaft 552. More specifically, the lead screw 555 can include a threaded outer surface 556 configured to engage/mate with the threaded portion 553 of the drive lumen 550. In the illustrated embodiment, the lead screw 555 includes a stop member 557 configured (e.g., sized and/or shaped) to engage the stop surface 551 in the third position shown in Figures 5C and 5F. The drive shaft 552 can be coupled to the lead screw 555 via welding, adhesives, fasteners, and/or other suitable types of connections, or the drive shaft 552 and the lead screw 555 can be manufactured from a single piece of wire or rod.

As shown in Figures 5D-5F, the hub assembly 436 can further include a lock pin 558 extending at least partially through the lock lumen 564 and operably coupling the inner hub component 540 to the outer hub component 542. For example, the lock pin 558 can include a flared portion 559 configured to engage a corresponding stop surface 560 of the outer hub component 542 or another component coupled to the outer hub component 542. The lock pin 558 further includes a stop member 561 configured to engage the stop surface 554 of the lock lumen 564 in the second position shown in Figures 5B and 5E. In the illustrated embodiment, the lock pin 558 defines a release lumen 563 configured to receive a release shaft 562. A dimension (e.g., diameter) of the release lumen 563 can be slightly greater than a corresponding dimension of the release shaft 562 such that an outer surface of the release shaft 562 engages an inner surface of the lock pin 558 when the release shaft 562 is positioned within the release lumen 563. In some embodiments, (i) the release shaft 562 is formed from a rigid material, such as a nickel-titanium alloy, that does not compress easily while (ii) the flared portion 559 of the lock pin 558 is biased radially inward and/or formed of a relatively compressible material. Accordingly, when the release shaft 562 is positioned in the release lumen 563, the release shaft 562 can bias/force the flared portion 559 of the lock pin 558 radially outward such that the flared portion 559 remains in engagement with the stop surface 560 of the outer hub component 542 even when the lock pin 558 is urged distally. In other embodiments, the hub assembly 436 can include other locking mechanisms for inhibiting movement of the inner hub component 540.

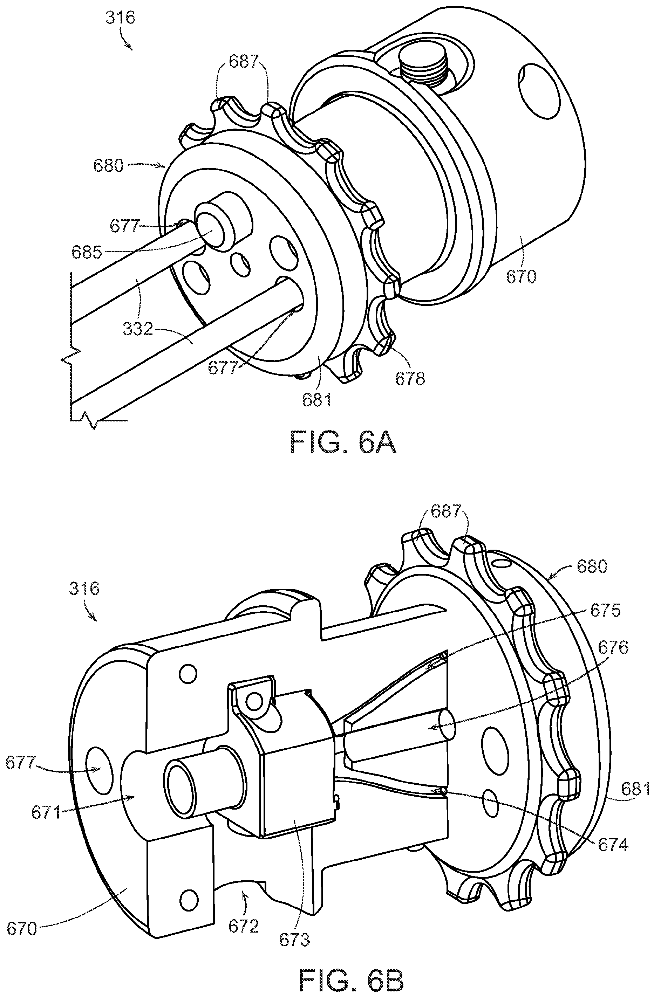

The drive shaft 552 and the release shaft 562 extend from the hub assembly 436, through the hub shaft 316 (Figure 4), and to the hub shaft handle 326. Figures 6A-6C are an isometric view, a partially cross-sectional proximally-facing isometric view, and a partially cross-sectional distally-facing isometric view, respectively, of the hub shaft handle 326 in accordance with embodiments of the present technology. In general, the hub shaft handle 326 can be manipulated/actuated by a user to actuate the drive shaft 552 and/or the release shaft 562 to drive movement of the hub assembly 436 and release the connectors 205 of the implantable device 200 (Figures 2A and 2B) during device deployment.

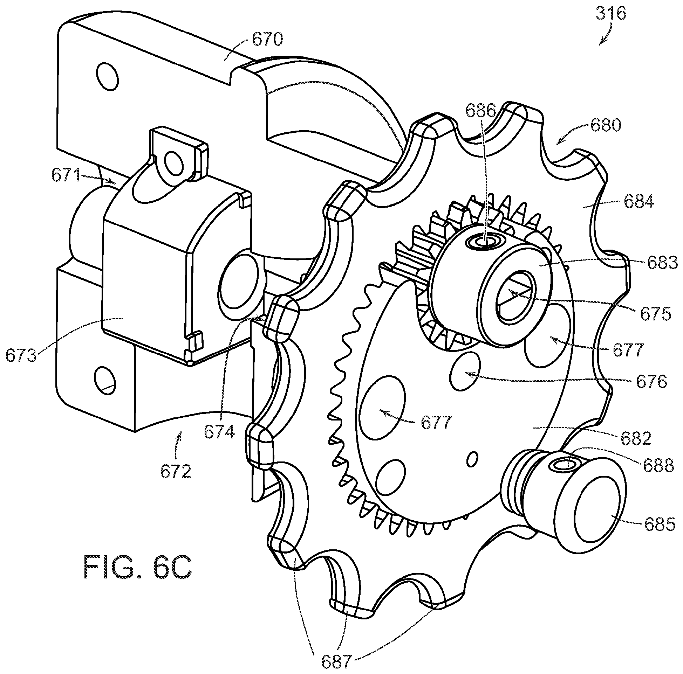

Referring to Figures 6A-6C together, the hub shaft handle 326 includes a body component 670 coupled to a gear assembly 680. The body component 670 is shown as cross-sectional in Figures 6B and 6C. The body component 670 includes/defines a series of interconnected lumens including a hub shaft lumen 671 and a valve lumen 672. The hub shaft handle 326 can further include a hub shaft connector 673 (Figures 6B and 6C) positioned within the body component 670. The hub shaft 316 (Figure 4; not shown in Figures 6A-6C for clarity) is configured to extend through the hub shaft lumen 671 and be secured to the hub shaft connector 673. The valve lumen 672 can receive one or more adaptors, valves, sealing members, etc., (not shown) that are configured for maintaining hemostasis and/or facilitating the ingress/egress of fluids (e.g., blood, priming solution, etc.) into the hub shaft 316. In some embodiments, for example, a duckbill hemostasis valve (not shown) is positioned in the valve lumen 672.

The gear assembly 680 includes a housing 681 (Figures 6A and 6B; omitted in Figure 6C for clarity) that can at least partially enclose the following components shown in Figure 6C: a body portion 682, a pinion gear 683, a ring gear 684, and a release member 685. The body component 670 and the gear assembly 680 (e.g., the housing 681, the body portion 682, and/or other components of the gear assembly 680) together include/define an additional series of interconnected lumens extending from the hub shaft connector 673 including (i) a release shaft lumen 674, (ii) a drive shaft lumen 675, (iii) a core shaft lumen 676, and (iv) one or more rail lumens 677. The core shaft 318 (Figure 3; not shown in Figures 6A-6C for clarity) extends through the hub shaft 316, the hub shaft connector 673, and the core shaft lumen 676 to the core shaft handle 328 (Figure 3). Accordingly, the core shaft 318 passes entirely through the hub shaft handle 326 and is independently movable relative to the hub shaft handle 326 and the hub shaft 316. The rail lumens 677 are configured to slidably receive the rails 332 (Figure 3; omitted in Figures 6B and 6C for clarity).

The drive shaft 552 (Figures 5D-5F; not shown in Figures 6A-6C for clarity) extends through the hub shaft 316, the hub shaft connector 673, and the drive shaft lumen 675, and is fixedly coupled to the pinion gear 683. Referring to Figure 6C, in some embodiments, the drive shaft 552 is secured to the pinion gear 683 via a set screw 686 and/or other suitable connection. The pinion gear 683 can be operably coupled to the ring gear 684 via, for example, the mating engagement of a plurality of teeth of the pinion gear 683 and the ring gear 684. The ring gear 684 is rotatable to rotate the pinion gear 683 to rotate the drive shaft 552. In some embodiments, the ring gear 684 includes a plurality of gripping features 687 around a perimeter thereof and that are accessible outside the housing 681. A user (e.g., a physician, other clinician, robotic or other automated mechanism), can manipulate the gripping features 687 to rotate the ring gear 684, thereby driving rotation of the drive shaft 552 to, for example, move the hub assembly 436 (Figures 5A-5F), as described in greater detail below. In some aspects of the present technology, the gripping features 687 accessible about its circumference to facilitate easy gripping and rotation of the ring gear 684. In some embodiments, the gear assembly 680 is configured to have a selected/selectable amount of frictional drag that inhibits rotation of the ring gear 684 to avoid rotation caused by inadvertent contact. These friction inducing features (e.g., compressed O-rings) and/or gear locks (not shown) operably coupled to the gear assembly 680 can avoid accidental rotation and promote only purposeful actuation of the gear assembly 680.

The release shaft 562 (Figures 5D-5F; not shown in Figures 6A-6C for clarity) extends through the hub shaft 316, the hub shaft connector 673, and the release shaft lumen 674, and is fixed to the release member 685 shown in Figure 6C. The release shaft 562 can be secured to the release member 685 via a set screw 688, other mechanical connector, adhesive, welding, or other suitable connection. The release member 685 can be releasably coupled the gear assembly 680 and configured to be moved (e.g., pulled proximally) away from the gear assembly 680 to move the release shaft 562 in a proximal direction through the hub shaft handle 326. In some embodiments, the release member 685 includes one or more locking features (e.g., one or more threaded connections, compressed O-rings, press-fittings) that must be disengaged to permit movement of the release member 685 to, for example, inhibit or even prevent accidental disengagement of the release member 685.

The operation of the hub shaft handle 326 and the hub assembly 436 for deploying/releasing the implantable device 200 is now described with reference to Figures 2A, 2B, and 4-6C. During device delivery to a target site, the hub assembly 436 is in the first position shown in Figures 5A and 5D such that all the connectors 205 of the implantable device 200 are secured/restrained in the first and second recesses 544, 546 by the outer hub component 542. When the implantable device 200 is at the desired target site (e.g., at or near the mitral valve), the user can initiate deployment by moving the hub assembly 436 to the second position shown in Figures 5B and 5E by actuating (e.g., rotating) the ring gear 684 of the hub shaft handle 326 and, thereby, rotating the drive shaft 552. Rotation of the drive shaft 552 rotates the lead screw 555, which drives (e.g., linearly translates) the inner hub component 540 toward and partially out of the distal opening 549 of the outer hub component 542 via the engagement of the threaded outer surface 556 of the lead screw 555 with the threaded portion 553 of the drive lumen 550. In the second position, at least a portion of each second recess 546 (e.g., the second portions 547 of the second recesses 546) is positioned sufficiently distal of the distal opening 549 of the outer hub component 542 that the connectors 205 positioned at the posterior side portion P of the implantable device 200 are no longer constrained by the outer hub component 542, and thus free to disengage from the hub assembly 436. While in the second position with the posteriorly-positioned connectors 205 released, the second portions 547 of the first recesses 544 remain covered by the outer hub component 542 such that the connectors 205 positioned at the anterior side portion A of the implantable device 200 remain constrained by the outer hub component 542. Accordingly, moving the hub assembly 436 to second position disengages the struts 206 at the posterior side portion P of the atrial-fixation member 202 from the hub assembly 436-allowing them to expand-while inhibiting the struts 206 at the anterior side portion A of the atrial-fixation member 202 from disengaging the hub assembly 436 and expanding. In some embodiments, the hub assembly 436 has other configurations that allow for selective disengagement of the connectors 205 in a different manner or order, such as the anteriorly-positioned connectors 205 releasing before the posteriorly-positioned connectors 205.

In some embodiments, in the second position, the lock pin 558 inhibits further distal movement of the inner hub component 540 (e.g., to the third position shown in Figures 5C and 5F). For example, the release shaft 562 can bias the flared portion 559 of the lock pin 558 radially outward and into engagement with the stop surface 560 of the outer hub component 542, while the stop member 561 engages the stop surface 554 of the lock lumen 564. In some aspects of the present technology, the lock pin 558 and the release shaft 562 inhibit or even prevent inadvertent deployment of the struts 206 at the anterior side portion A of the atrial-fixation member 202 by inhibiting the first recesses 544 from being moved distally past the distal opening 549 of the outer hub component 542 (e.g., to the third position shown in Figures 5C and 5F).

To move the move the hub assembly 436 to the third position shown in Figures 5C and 5F, the user can first remove the release shaft 562 from the release lumen 563 of the lock pin 558 by moving (e.g., pulling) the release member 685 proximally away from the gear assembly 680 of the hub shaft handle 326. The user can then further actuate the ring gear 684 of the hub shaft handle 326 to drive the drive shaft 552 to rotate. Removing the release shaft 562 removes the outward biasing force provided by the release shaft 562 when the flared portion 559 is urged inward, and thereby permits the flared portion 559 of the lock pin 558 to move (e.g., flex) radially inward and past the stop surface 560 of the outer hub component 542 when the drive shaft 552 and the lead screw 555 drive the inner hub component 540 distally. In the third position, the first recesses 544 (e.g., the second portions 547 of the first recesses 544) are positioned sufficiently distal of the distal opening 549 of the outer hub component 542 that the connectors 205 positioned at the anterior side portion A of the implantable device 200 are no longer constrained by the outer hub component 542, and are thus free to disengage the hub assembly 436. Accordingly, moving the hub assembly 436 to third position disengages the struts 206 at the anterior side portion A of the atrial-fixation member 202 from the hub assembly 436, allowing them to expand. Therefore, in some aspects of the present technology the hub assembly 436 is configured to provide for a controlled, two-stage release of the atrial-fixation member 202 in which the struts 206 at the posterior side portion P are released from the hub assembly 436 and allowed to expand before the struts 206 at the anterior side portion A. In additional aspects of the present technology, the hub assembly 436 allows for the controlled and staged release of the atrial-fixation member 202 from the distal end of the delivery system 310 (Figure 3) and is not affected or minimally affected by factors such as catheter shaft compression, curving, and the like that could make it difficult to control the release of the atrial-fixation member 202 with the same precision via control from the proximal end of the delivery system 310.

Figures 7A and 7B are a partially-transparent isometric side view and an enlarged partially cross-sectional side view, respectively, of the plug assembly 438 at the distal end portion of the delivery system 310 of Figures 3 and 4 in accordance with embodiments of the present technology. Referring to Figures 7A and 7B together, the plug assembly 438 includes a plug housing 790 configured to be secured to/at a distal terminus of the core shaft 318. The plug housing 790 includes a proximal end portion 791a and a distal end portion 791b. In some embodiments, a section of the plug housing 790 extends at least partially into a lumen 792 defined by the core shaft 318 and can be secured thereto via a friction-fit arrangement, adhesive, laser-weld, fasteners, and/or other suitable attachment mechanisms.

The plug assembly 438 further includes/defines a plurality of lumens including a drive lumen 793, a clip tendon lumen 794, and a cinch tendon lumen 795 (collectively "lumens 793-795"). In the illustrated embodiment, the lumens 793-795 are defined by tubes coupled to the plug housing 790 and that extend distally into the lumen 792 of the core shaft 318. In some embodiments, the tubes are short, flexible tubes while, in other embodiments the tubes can be rigid or semi-rigid and/or can extend through the entire length of the core shaft 318. In other embodiments, the lumens 793-795 can be defined solely within the plug housing 790 and/or can extend through a wall of the core shaft 318. For example, the plug housing 790 can be a single-piece, such as a plastic extrusion, that defines each of the lumens 793-795.

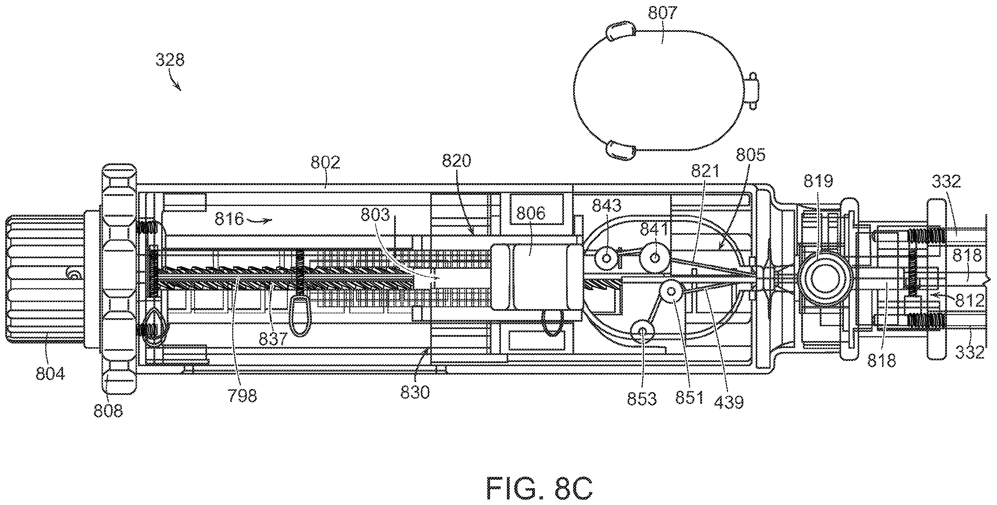

The clip tendon lumen 794 is configured to receive a first elongated flexible element, such as a suture, string, and/or a wire (e.g., a clip tendon 821 shown in Figure 8C) for actuating the clip 209 of the baffle 204 (Figures 2A and 2B), as described in further detail below. The cinch tendon lumen 795 extends distally to the flexible tube 437 and is configured to receive a second elongated flexible element, such as a cinch tendon 439 (Figure 4) for controlling expansion and contraction of the atrial-fixation member 202. In some embodiments, one or both of the first and second elongated flexible elements (e.g., the clip and cinch tendons) can extend from the proximal portion of the delivery system 310 (Figure 3) to the distal end portion, where they form a loop around an element (e.g., connection features on the clip 209 (Figure 2A) and/or the atrial-fixation member 202 (Figures 2A and 2B)), and then extend back up through the delivery system 310 to the proximal end (e.g., referred to as "double-length suture loops"). In these embodiments, the clip tendon lumen 794 and/or the cinch tendon lumen 795 carry two sections of the elongated flexible elements, and in certain embodiments the tubes carrying the flexible elements divide the lumens 794, 795 into separate channels that carry the individual sections of the elongated flexible members to avoid entanglement or excessive friction.

In the illustrated embodiment, the plug assembly 438 includes a baffle connection member 796, such as a threaded shaft (e.g., a screw), secured to the plug housing 790 in a distal portion of the drive lumen 793 and extending out of the drive lumen 793 beyond the distal end portion 791b of the plug housing 790. In some embodiments, the baffle connection member 796 is rotatably coupled within the drive lumen 793. With additional reference to Figure 4, the baffle connection member 796 is configured to extend through the opening 201 in the baffle 204 to releasably engage the delivery attachment member 203 (e.g., via a threaded connection) to secure the core shaft 318 to the baffle 204 of the implantable device 200. A drive shaft 798 can extend through the drive lumen 793 to operably couple the baffle connection member 796 to an actuation means (e.g., a wheel, knob, button) at the proximal portion of the delivery system 310 (Figure 3), which can be actuated to impart rotation on the baffle connection member 796, thereby disengaging the baffle connection member 796 from the delivery attachment member 203. The drive shaft 798 can be coupled to the baffle connection member 796 via welding, adhesives, fasteners, and/or other suitable types of connections, or be manufactured from a single piece of wire.

The clip tendon, the cinch tendon 439, and the drive shaft 798 extend from the plug assembly 438, through the core shaft 318, and to the core shaft handle 328. Figures 8A-8D are a distally-facing isometric view, a partially transparent side view, a partially transparent top view, and a partially transparent proximally-facing isometric view, respectively, of the core shaft handle 328 configured in accordance with embodiments of the present technology. In general, the core shaft handle 328 is configured to be manipulated/actuated by a user to individually actuate (i) the clip tendon to open/close the clip 209 (Figures 2A and 2B), (ii) the drive shaft 798 to unscrew the baffle connection member 796 (Figures 7A and 7B) from the baffle 204 (Figures 2A and 2B), and (iii) the cinch tendon 439 to cinch/uncinch the atrial-fixation member 202 (Figures 2A and 2B).

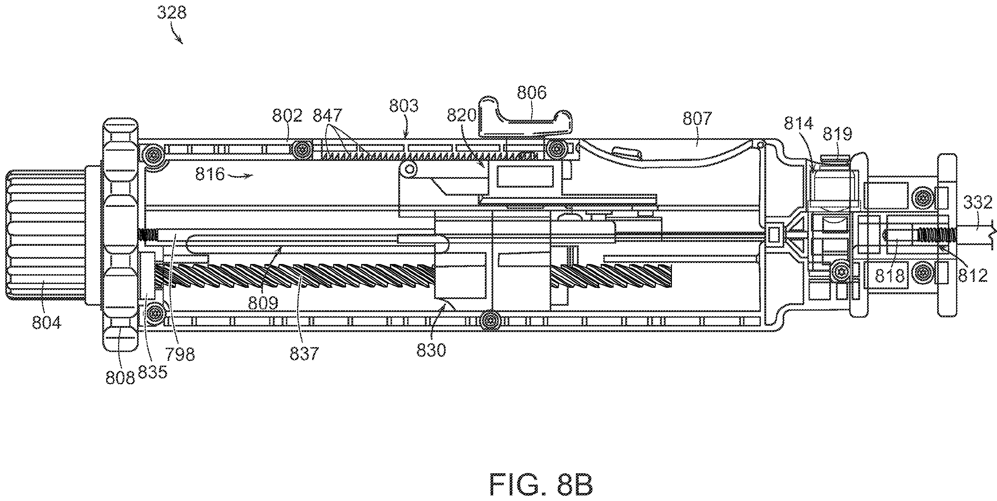

Referring to Figures 8A-8D together, the core shaft handle 328 includes a core shaft housing 802 and a plurality of actuators coupled to the core shaft housing 802, including, for example, a baffle actuator 804, a clip actuator 806, and a cinch actuator 808. The housing 802 is shown as partially transparent in Figures 8B-8D. The housing 802 and/or other components of the core shaft handle 328 define a series of interconnected lumens including a core shaft lumen 812, a valve lumen 814, and an actuation lumen 816 (collectively "lumens 812-816"). The core shaft handle 328 further includes a core shaft connector 818 positioned between the lumens 812-816. The core shaft 318 is configured to extend through the core shaft lumen 812 and be secured to the core shaft connector 818. The valve lumen 814 is configured to receive a valve 819 and/or one or more additional adaptors, valves, sealing members, and/or other fluid control components for, for example, maintaining hemostasis, facilitating the ingress/egress of fluids (e.g., blood, priming solution, saline) into the core shaft 318, etc. The rails 332 can be secured to the housing 802 and/or other components of the core shaft handle 328 to slidably couple the core shaft handle 328 to the hub shaft handle 326 (Figure 3).

The drive shaft 798 can extend through the core shaft 318, the core shaft connector 818, and into the actuation lumen 816 where it is secured to the baffle actuator 804. The baffle actuator 804 is accessible to a user via the housing 802 at a proximal portion of the core shaft handle 328 and is actuatable to drive the drive shaft 798. In the illustrated embodiment, for example, the baffle actuator 804 can be rotated to rotate the drive shaft 798. Accordingly, with additional reference to Figures 4, 7A, and 7B, a user (e.g., a physician) can grip and rotate the baffle actuator 804 to impart rotation onto the drive shaft 798, which in turn rotates the baffle connection member 796 to disengage the baffle connection member 796 from the delivery attachment member 203 and, thereby, disengages/detaches the baffle 204 from the core shaft 318.

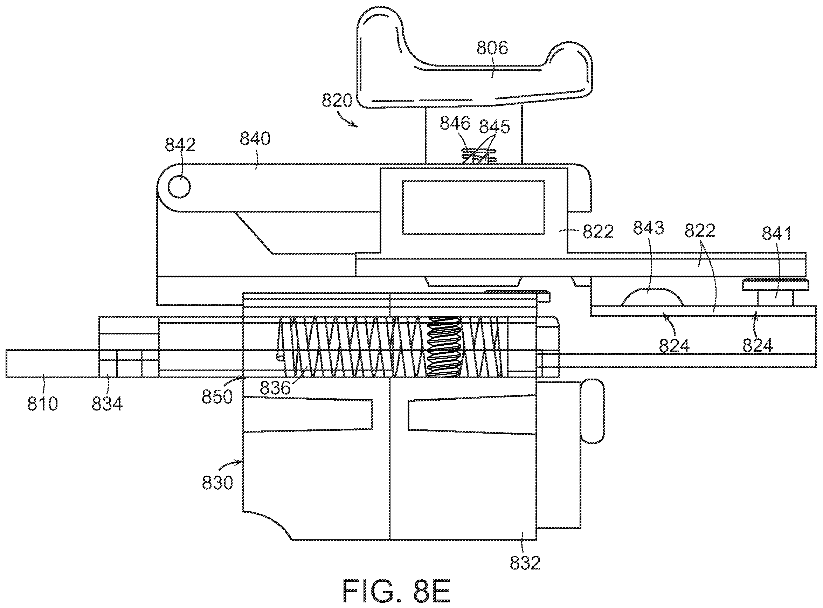

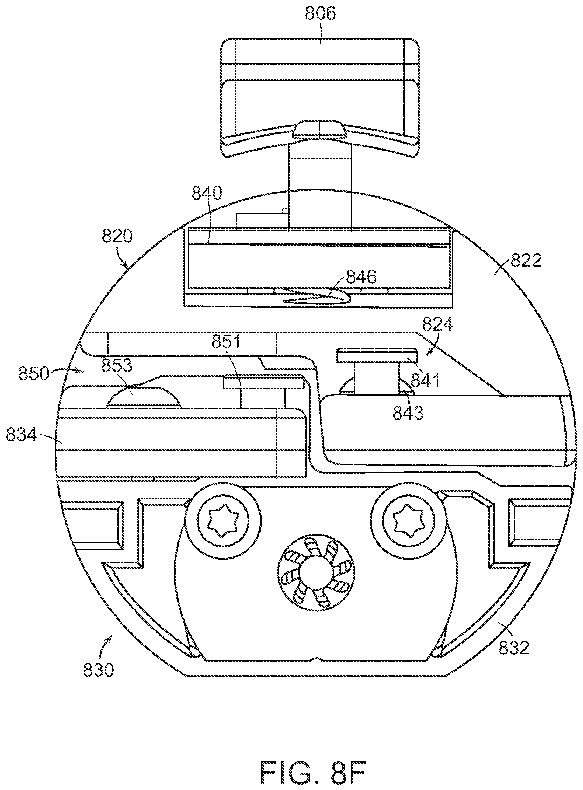

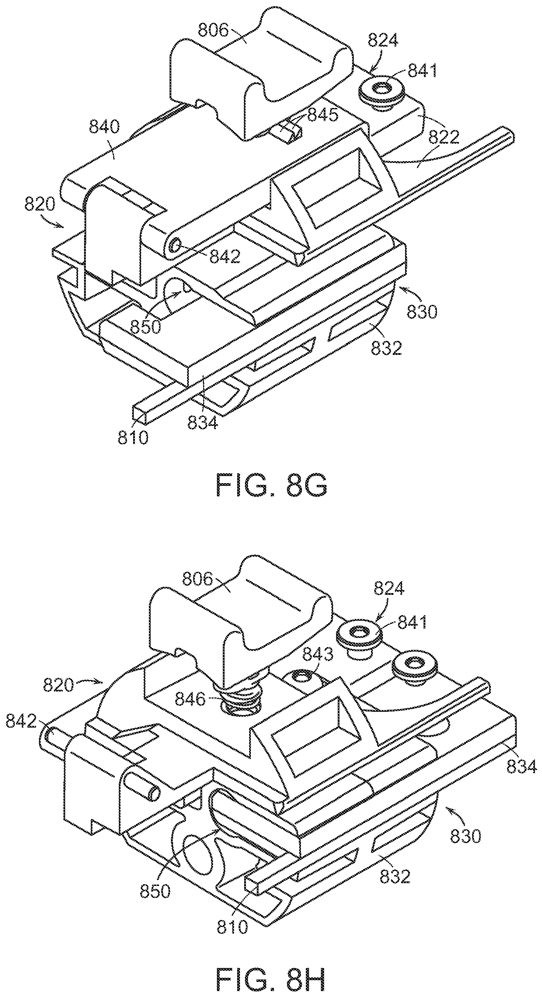

In the illustrated embodiment, the core shaft handle 328 includes a clip mount assembly 820 and a cinch mount assembly 830 slidably positioned within the actuation lumen 816. The clip mount assembly 820 is operably coupled to the clip actuator 806 and the cinch mount assembly 830 is operably coupled to the cinch actuator 808. Figures 8E-8H are a partially transparent side view, a proximally-facing front view, a distally-facing isometric view, and another distally-facing isometric view, respectively, of the clip mount assembly 820 and the cinch mount assembly 830 configured in accordance with embodiments of the present technology. The cinch mount assembly 830 is in (i) a relaxed (e.g., first, non-tensioned, minimally-tensioned) configuration in Figures 8E and 8G and (ii) a tensioned (e.g., second) configuration in Figures 8F and 8H.

Referring to Figures 8E-8H together, the clip mount assembly 820 can include a body 822, a clip tendon mount 824 fixed to the body 822, and a latch 840 movably (e.g., pivotally) coupled to the body 822 via, for example, a shaft 842. The latch 840 is omitted in Figure 8H for clarity. The clip actuator 806 can be coupled to the latch 840 and slidably mounted to a first slot 803 (Figures 8A-8D) extending through/along the housing 802. With additional reference to Figure 8C, the clip tendon (e.g., a clip tendon 821 shown in Figure 8C) extends through the core shaft 318, the core shaft connector 818, and into the actuation lumen 816 where it is releasably coupled to the clip tendon mount 824. In the illustrated embodiment, for example, the clip tendon mount 824 comprises a post 841 and a screw 843. The clip tendon 821 can be a double-length suture loop that is wound around the post 841 (e.g., one time, two times, ten times, more than ten times) and then secured to the body 822 via the screw 843. As best seen in Figures 8E and 8H, the latch 840 can include a plurality of first engagement features 845 (e.g., teeth, grooves) and can be operably coupled to the body 822 via a biasing member 846, such as a compression spring. As best seen in Figures 8A and 8B, the housing 802 can include a plurality of second engagement features 847 (e.g., teeth, grooves) positioned adjacent to the first slot 803 within the actuation lumen 816. Referring to Figures 8A, 8B, 8E, and 8H together, the biasing member 846 can normally bias the first engagement features 845 into engagement with the second engagement features 847 to inhibit movement of the clip actuator 806 and the clip mount assembly 820 along the first slot 803. To move the clip mount assembly 820 through the actuation lumen 816, a user can depress the clip actuator 806 against the biasing force of the biasing member 846 to disengage the first engagement features 845 from the second engagement features 847 and then slide the clip actuator 806 along the first slot 803.

Referring again to Figures 8A-8D together, during a delivery procedure, actuation of the clip actuator 806 can drive the clip mount assembly 820 within the actuation lumen 816 to drive/tension the clip tendon 821 to open/close the clip 209 (Figure 2B). More specifically, Figures 8A-8D illustrate the clip actuator 806 in a first position in which the clip actuator 806 is positioned proximate a distal end portion of the first slot 803. In operation, the user can depress the clip actuator 806 and slide the clip actuator 806 along the first slot 803 toward a second position proximate a proximal end portion of the first slot 803 (e.g., in a direction indicated by arrow P in Figure 8A). This proximal movement of the clip actuator 806 can pull the clip tendon 821 to open the clip 209. Likewise, distal movement of the clip actuator 806 can release the force/tension on the clip tendon 821, thereby allowing the normally-closed clip 209 to close. In some embodiments, the first slot 803 can have a length selected to correspond to a specific opening/closing stroke of the clip 209. For example, a position of the distal end portion of the first slot 803 can be selected to correspond to a fully-closed position of the clip 209 and/or a position of the proximal end portion of the first slot 803 can be selected to correspond to a fully-open position of the clip 209. In other embodiments, the core shaft handle 328 can include other features for actuating the clip tendon 821 to open/close the clip 209, such as one or more buttons, levers, knobs, sliders, and/or other actuation members.

Referring again to Figures 8E-8H together, the cinch mount assembly 830 can include a body 832, a cinch tendon mount 834 (shown partially transparent in Figure 8E for clarity), and a biasing member 836 (Figure 8E) operably coupling the cinch tendon mount 834 to the body 832. In the illustrated embodiment, the body defines a channel 850 and the cinch tendon mount 834 is slidably mounted within the channel 850. With additional reference to Figure 8C, the cinch tendon 439 (Figure 8C) extends through the core shaft 318, the core shaft connector 818, and into the actuation lumen 816 where it is coupled to the cinch tendon mount 834. In the illustrated embodiment, for example, the clip tendon mount 824 includes a post 851 and a screw 853 (both obscured in Figures 8E and 8H), and the cinch tendon 439 can be wound around the post 851 and further secured (e.g., clamped) via the screw 853.

Referring again to Figures 8A-8D together, the cinch actuator 808 can be a ring gear that is accessible from the housing 802 for actuation by a user. The cinch actuator 808 can be operably coupled to the cinch mount assembly 830 via a pinion gear 835 and a lead screw 837. More specifically, the pinion gear 835 can be coupled to the cinch actuator 808 via, for example, the mating engagement of a plurality of teeth of the pinion gear 835 and the cinch actuator 808. The pinion gear 835 can be fixedly mounted to the lead screw 837, which can threadedly engage the cinch mount assembly 830 (e.g., the body 832). Accordingly, with additional reference to Figure 4, the user can grip and rotate the cinch actuator 808 to (i) drive the lead screw 837 to rotate, (ii) drive the cinch mount assembly 830 to move (e.g., translate) within the actuation lumen 816, and (iii) drive/tension the cinch tendon 439 to cinch/uncinch the atrial-fixation member 202 of the implantable device 200 (Figures 2A and 2B). For example, the cinch mount assembly 830 is in a first position in Figures 8A-8D in which the cinch mount assembly 830 is positioned proximate a distal portion of the actuation lumen 816. In operation, rotation of the cinch actuator 808 in a first direction can drive the cinch mount assembly 830 proximally through the actuation lumen 816 to pull the cinch tendon 439 proximally to radially compress the atrial-fixation member 202. Conversely, rotation of the cinch actuator 808 in a second direction can drive the cinch mount assembly 830 distally through the actuation lumen 816 to relax the cinch tendon 439 to permit the atrial-fixation member 202 to radially expand.

Referring to Figures 8A-8H together, in some embodiments the biasing member 836 stores energy (e.g., becomes "loaded") as the cinch mount assembly 830 is driven proximally through the actuation lumen 816. In some embodiments, the biasing member 836 can be a compression spring that compresses due to the opposing forces between the body 832 (e.g., via the lead screw 837) and the cinch tendon mount 834 (e.g., via the cinch tendon 439). In the illustrated embodiment, the cinch tendon mount 834 includes a cinch indicator portion 810 that can be viewed through, for example, a second slot 809 extending through/along the housing 802. A distance between the cinch indicator portion 810 of the cinch tendon mount 834 and the body 832 can indicate a relative amount of cinching. For example, in the relaxed position shown in Figures 8E and 8G, the biasing member 836 can bias the cinch tendon mount 834 away from the body 832-increasing the distance between the cinch indicator portion 810 and the body 832. Conversely, in the tensioned position shown in Figures 8F and 8H, the cinching forces can compress the biasing member 836-decreasing the distance between the cinch indicator portion 810 and the body 832. Accordingly, the user can view the positioning of the cinch indicator portion 810 along the second slot 809 to determine an amount of cinching.

In additional aspects of the present technology, the biasing member 836 of the cinch mount assembly 830 is configured to spring-load the cinch tendon 439 such that tension is maintained on the cinch tendon 439 during manipulation of the delivery system 310. With additional reference to Figures 2A and 2B, this can inhibit or even prevent unwanted cinching/uncinching of the atrial-fixation member 202. In some embodiments, for example, actuating the cinch actuator 808 to cinch the implantable device 200 can load the biasing member 836 such that the biasing member 836 can take up any slack in the cinch tendon 439 during manipulation of the delivery system 310. In other embodiments, the core shaft handle 328 can include other features for actuating the cinch tendon 439 to cinch/uncinch the implantable device 200, such as one or more buttons, levers, knobs, sliders, and/or other actuators. For example, in some embodiments the cinch tendon 439 can be actuated via the same or a similar arrangement as the clip tendon 821 (e.g., via sliding movement of the cinch actuator 808 along the housing 802).

In some embodiments, the clip tendon mount 824 of the clip mount assembly 820 and the cinch tendon mount 834 of the cinch mount assembly 830 are each nearly aligned with a longitudinal axis/centerline of the core shaft handle 328. This arrangement can help ensure that the tension/drive forces on the cinch tendon 439 and the clip tendon 821 (collectively "tendons 439, 821") are relatively aligned along the axes of the tendons 439, 821 and help inhibit shear or other forces that could prematurely break the tendons 439, 821. In some aspects of the present technology, the clip mount assembly 820 and the cinch mount assembly 830 are independently movable through the actuation lumen 816. For example, in the illustrated embodiment the body 822 of the clip mount assembly 820 and the body 832 of the cinch mount assembly 830 each have complementary shapes (e.g., generally L-shapes) that allow the clip and cinch mount assemblies 820, 830 to move through the actuation lumen 816 without interfering with one another. In other embodiments, the clip and cinch mount assemblies 820, 830 can be operably coupled to move together.

Referring to Figure 8C, the housing 802 can further include an opening 805 configured (e.g., shaped, sized, and/or positioned) to provide access to the tendons 439, 821. In operation, for example, the user can remove the tendons 439, 821 by (i) cutting the tendons 439, 821 by inserting a cutting tool through the opening 805 and (ii) pulling the tendons 439, 821 out of the opening 805. In other embodiments, the core shaft handle 328 can have other features for cutting and/or removing the tendons 439, 821. In some embodiments, the housing 802 further includes a removable cover 807 that can be releasably secured over the opening 805 (e.g., via a snap-fit arrangement) to conceal the tendons 439, 821 and enclose the internal features the core shaft handle 328.

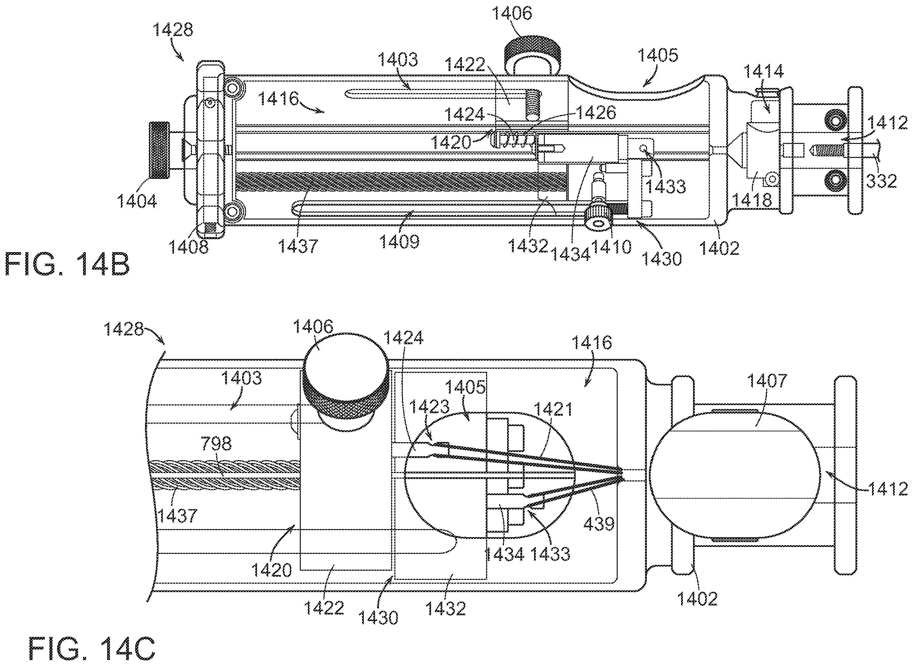

Figures 14A-14D are a distally-facing isometric view, a partially transparent side view, a partially transparent enlarged side view, and a partially transparent proximally-facing isometric view, respectively, of a core shaft handle 1428 configured in accordance with additional embodiments of the present technology. The core shaft handle 1428 can (i) include several features generally similar or identical to the core shaft handle 328 described in detail above with reference to Figures 8A-8H, (ii) operate generally similarly or identically to the core shaft handle 328, and/or (iii) can be integrated into the system 310 in a generally similar or identical manner. Referring to Figures 14A-14D together, the core shaft handle 1428 includes a core shaft housing 1402 and a plurality of actuators coupled to the core shaft housing 1402, including, for example, a baffle actuator 1404, a clip actuator 1406, and a cinch actuator 1408. The housing 1402 and/or other components of the core shaft handle 1428 define a series of interconnected lumens including a core shaft lumen 1412, a valve lumen 1414, and an actuation lumen 1416 (collectively "lumens 1412-1416"). As best seen in Figure 14B, the core shaft handle 1428 further includes a core shaft connector 1418 positioned between the lumens 1412-1416. The core shaft 318 (Figure 3; not shown in Figures 14A-8D for the sake of clarity) is configured to extend through the core shaft lumen 1412 and be secured to the core shaft connector 1418. The valve lumen 1414 is configured to receive one or more adaptors, valves, sealing members, and/or other fluid control components (not shown) for, for example, maintaining hemostasis, facilitating the ingress/egress of fluids (e.g., blood, priming solution, saline) into the core shaft 318, and the like. As best seen in Figures 14A and 14B, the rails 332 can be secured to the housing 1402 and/or other components of the core shaft handle 1428 to slidably couple the core shaft handle 1428 to the hub shaft handle 326.

The drive shaft 798 can extend through the core shaft 318, the core shaft connector 1418, and into the actuation lumen 1416 where it is secured to the baffle actuator 1404. The baffle actuator 1404 is accessible to a user via the housing 1402 at a proximal portion of the core shaft handle 1428 and is actuatable to drive the drive shaft 798. In the illustrated embodiment, for example, the baffle actuator 1404 can be rotated to rotate the drive shaft 798. Accordingly, with additional reference to Figures 4, 7A, and 7B, a user (e.g., a physician) can grip and rotate the baffle actuator 1404 to impart rotation onto the drive shaft 798, which in turn rotates the baffle connection member 796 to disengage the baffle connection member 796 from the delivery attachment member 203 and, thereby, disengages/detaches the baffle 204 from the core shaft 318.

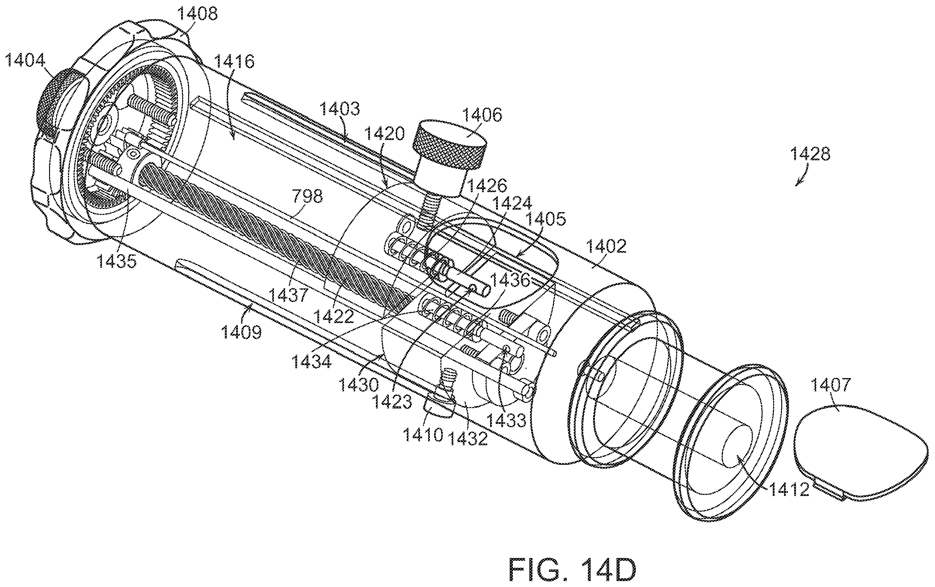

In the illustrated embodiment, the core shaft handle 1428 includes a clip mount assembly 1420 slidably positioned within the actuation lumen 1416. The clip tendon (e.g., a clip tendon 1421 shown in Figure 14C) extends through the core shaft 318, the core shaft connector 1418, and into the actuation lumen 1416 where it is releasably coupled to the clip mount assembly 1420. In some embodiments, the clip mount assembly 1420 includes a body 1422, a clip tendon mount 1424, and a biasing member 1426 operably coupling the clip tendon mount 1424 to the body 1422. The body 1422 is shown as partially transparent in Figures 14B and 14D for clarity. The clip actuator 1406 can be slidably mounted to a first slot 1403 extending through/along the housing 1402, and is operably coupled to the clip mount assembly 1420 through the first slot 1403. In the illustrated embodiment, the clip tendon 1421 is a double-length suture loop that extends through an eyelet 1423 in the clip tendon mount 1424.

During a delivery procedure, actuation of the clip actuator 1406 can drive the clip mount assembly 1420 within the actuation lumen 1416 to drive/tension the clip tendon 1421 to open/close the clip 209 (Figure 2B). More specifically, Figures 14A-14D illustrate the clip actuator 1406 in a first position in which the clip actuator 1406 is positioned proximate a distal end portion of the first slot 1403. In operation, the user can grip the clip actuator 1406 and slide the clip actuator 1406 along the first slot 1403 toward a second position proximate a proximal end portion of the first slot 1403 (e.g., in a direction indicated by arrow P in Figure 14A). This proximal movement of the clip actuator 1406 can pull the clip tendon 1421 to open the clip 209. Likewise, distal movement of the clip actuator 1406 can release the force/tension on the clip tendon 1421, thereby allowing the normally-closed clip 209 to close. In some embodiments, the first slot 1403 can have a length selected to correspond to a specific opening/closing stroke of the clip 209. For example, a position of the distal end portion of the first slot 1403 can be selected to correspond to a fully-closed position of the clip 209 and/or a position of the proximal end portion of the first slot 1403 can be selected to correspond to a fully-open position of the clip 209. In some embodiments, the biasing member 1426 stores energy (e.g., becomes "loaded") as the clip mount assembly 1420 is driven proximally through the actuation lumen 1416. In various embodiments, the core shaft handle 1428 can include other features for actuating the clip tendon 1421 to open/close the clip 209, such as one or more buttons, levers, knobs, sliders, and/or other actuation members.

As further shown in Figures 14B-14D, the core shaft handle 1428 can also include a cinch mount assembly 1430 slidably positioned within the actuation lumen 1416. The cinch mount assembly 1430 can include features generally similar or identical to those of the clip mount assembly 1420. In the illustrated embodiment, for example, the cinch mount assembly 1430 includes a body 1432 (shown partially transparent in Figures 14B and 14D for clarity), a cinch tendon mount 1434, and a biasing member 1436 operably coupling the cinch tendon mount 1434 to the body 1432. The cinch tendon 439 extends through the core shaft 318, the core shaft connector 1418, and into the actuation lumen 1416 where it is coupled to the cinch tendon mount 1434 via an eyelet 1433 formed therein.

In some embodiments, as best seen in Figure 14D, the cinch actuator 1408 is a ring gear that is accessible from the housing 1402 for actuation by a user. The cinch actuator 1408 can be operably coupled to the cinch mount assembly 1430 via a pinion gear 1435 and a lead screw 1437. More specifically, the pinion gear 1435 can be coupled to the cinch actuator 1408 via, for example, the mating engagement of a plurality of teeth of the pinion gear 1435 and the cinch actuator 1408. The pinion gear 1435 can be fixedly mounted to the lead screw 1437, which can threadedly engage the cinch mount assembly 1430 (e.g., the body 1432). Accordingly, with additional reference to Figure 4, the user can grip and rotate the cinch actuator 1408 to (i) drive the lead screw 1437 to rotate, (ii) drive the cinch mount assembly 1430 to move (e.g., translate) within the actuation lumen 1416, and (iii) drive/tension the cinch tendon 439 to cinch/uncinch the atrial-fixation member 202 of the implantable device 200. For example, the cinch mount assembly 1430 is in a first position in Figures 14A-14D in which the cinch mount assembly 1430 is positioned proximate a distal portion of the actuation lumen 1416. In operation, rotation of the cinch actuator 1408 in a first direction can drive the cinch mount assembly 1430 proximally through the actuation lumen 1416 to pull the cinch tendon 439 proximally to radially compress the atrial-fixation member 202 (Figures 2A and 2B). Conversely, rotation of the cinch actuator 1408 in a second direction can drive the cinch mount assembly 1430 distally through the actuation lumen 1416 to relax the cinch tendon 439 to permit the atrial-fixation member 202 to radially expand. In some embodiments, the biasing member 1436 stores energy (e.g., becomes "loaded") as the cinch mount assembly 1430 is driven proximally through the actuation lumen 1416.

In various embodiments, the core shaft handle 1428 can include other features for actuating the cinch tendon 439 to cinch/uncinch the implantable device 200, such as one or more buttons, levers, knobs, sliders, and/or other actuators. For example, in some embodiments the cinch tendon 439 can be actuated via the same or a similar arrangement as the clip tendon 1421 (e.g., via sliding movement of the cinch actuator 1408 along the housing 1402).

In some aspects of the present technology, the clip mount assembly 1420 and the cinch mount assembly 1430 are independently movable through the actuation lumen 1416. For example, in the illustrated embodiment the body 1422 of the clip mount assembly 1420 and the body 1432 of the cinch mount assembly 1430 each have complementary shapes (e.g., semi-circular cross-sectional shapes) that allow the clip and cinch mount assemblies 1420, 1430 to move through the actuation lumen 1416 without interfering with one another. In other embodiments, the clip and cinch mount assemblies 1420, 1430 can be operably coupled to move together.

In another aspect of the present technology, the biasing member 1426 of the clip mount assembly 1420 and the biasing member 1436 of the cinch mount assembly 1430 are configured to spring-load the clip tendon 1421 and the cinch tendon 439 (collectively "tendons 439, 1421"), respectively, such that tension is maintained on the tendons during manipulation of the delivery system 310. With additional reference to Figures 2A and 2B, this can inhibit or even prevent unwanted actuation of the clip 209 or cinching/uncinching of the atrial-fixation member 202. In some embodiments, for example, actuating the cinch actuator 1408 to cinch the implantable device 200 can load the biasing member 1436 such that the biasing member 1436 can take up any slack in the cinch tendon 439 during manipulation of the delivery system 310. In another aspect of the present technology, the clip tendon mount 1424 of the clip mount assembly 1420 and the cinch tendon mount 1434 of the cinch mount assembly 1430 are each nearly aligned with a longitudinal axis/centerline of the core shaft handle 1428. This arrangement can help ensure that the tension/drive forces on the tendons 439, 1421 are relatively aligned along the axes of the tendons 439, 1421 and help inhibit shear or other forces that could prematurely break the tendons 439, 1421.

Referring to Figures 14B and 14D together, the housing 1402 can further include an opening 1405 configured (e.g., shaped, sized, and/or positioned) to provide access to the tendons 439, 1421. In operation, for example, the user can remove the tendons 439, 1421 by (i) cutting the tendons 439, 1421 by inserting a cutting tool through the opening 1405 and (ii) pulling the tendons 439, 1421 out of the opening 1405. In other embodiments, the core shaft handle 1428 can have other features for cutting/removing the tendons 439, 1421. In some embodiments, the housing 1402 further includes a removable cover 1407 (Figures 14A, 14C, and 14D) that can be releasably secured over the opening 1405 (e.g., via a snap-fit arrangement) to conceal the tendons 439, 1421 and enclose the internal features the core shaft handle 1428.

In the illustrated embodiment, a cinch indicator 1410 is coupled to the cinch mount assembly 1430 and extends at least partially out of a second slot 1409 extending through/along the housing 1402. For example, the cinch indicator 1410 can be coupled to the body 1432 of the cinch mount assembly 1430 via a threaded fastener or other suitable fastener. In operation, the cinch indicator 1410 moves along the second slot 1409 as the cinch actuator 1408 drives movement of the cinch mount assembly 1430 through the actuation lumen 1416. The user can view the position of the cinch indicator 1410 along the second slot 1409 to determine an amount of cinching. In some embodiments, the second slot 1409 can have a length selected to provide a maximum/minimum amount of cinching of the atrial-fixation member 202. For example, a position of a distal end portion of the second slot 1409 can be selected to correspond to a minimum amount of cinching and/or a position of a proximal end portion of the second slot 1409 can be selected to correspond to a maximum amount of cinching. In some embodiments, the cinch indicator 1410 can inhibit or even prevent rotation of the cinch mount assembly 1430 within the actuation lumen 1416.

Referring to Figures 3-8H and 13A-14D together, the various components of the delivery system 310 can be formed from metals (e.g., stainless steel, nickel-titanium alloys, etc.), plastics, and/or other suitable materials. The various components can be manufactured using three-dimensional printing, injection molding, machining, and/or other suitable processes known in the art. Moreover, the various means of actuation can be combined or changed without deviating from the scope of the present technology.

### III. Selected Embodiments of Methods of Delivering Implantable Devices

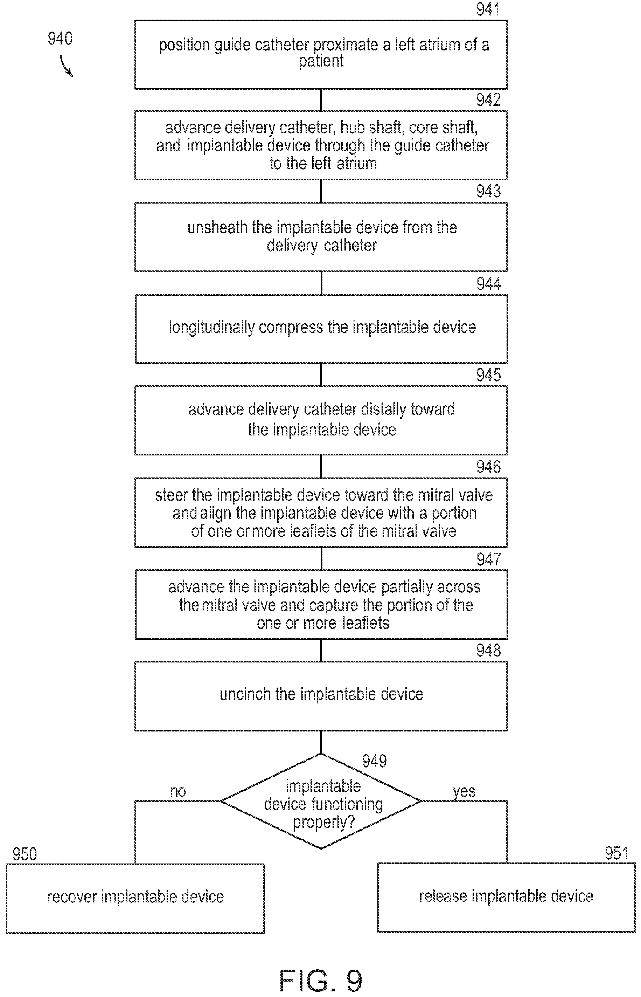

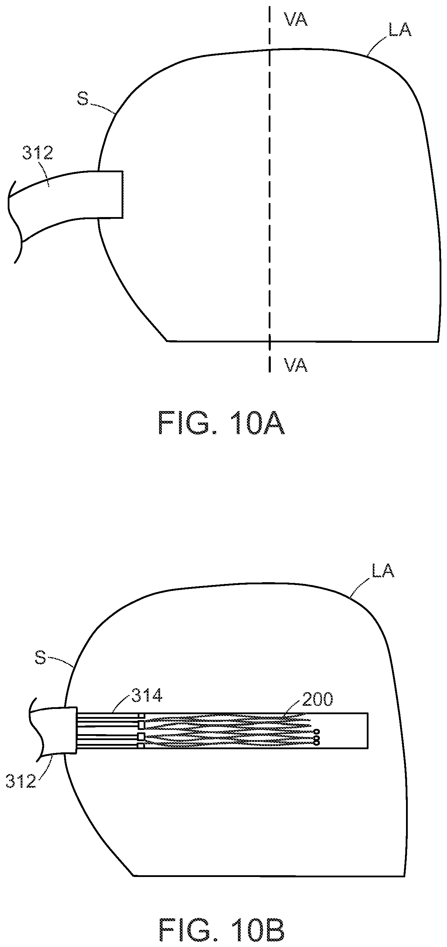

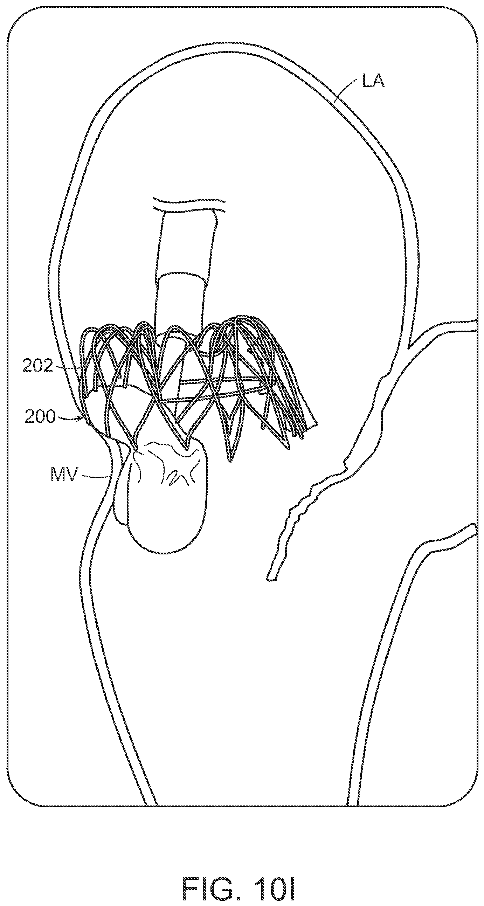

Figure 9 is a flow diagram of a process or method 940 for operating the delivery system 310 (Figures 3A-8D, 13A, and 13B) to implant the implantable device 200 (Figures 2A, 2B and 4) at a target site within a patient (e.g., at a native mitral valve of a human patient) in accordance with embodiments of the present technology. Figures 10A-10I are side views illustrating the implantable device 200 and a distal portion of the delivery system 310 during various stages of the method 940 in accordance with embodiments of the present technology. Although some features of the method 940 are described in the context of the embodiments shown in Figures 2A-8H, 13A, and 13B for the sake of illustration, one skilled in the art will readily understand that the method 900 can be carried out using other suitable systems and/or devices described herein (e.g., including the core shaft handle 1428 described in detail with reference to Figures 14A-14D). Likewise, although the method 940 is described in the context of delivering the implantable device to a native mitral valve, the method 940 can be employed to deliver implantable devices to other locations (e.g., to other cardiac valves) of a patient.

At block 941, the method 940 includes positioning the guide catheter 312 proximate a left atrium of a patient. For example, referring to Figure 10A, the guide catheter 312 can be inserted to traverse the venous system (e.g., via femoral or axillary access) to the right atrium and then across the interatrial septum S into the left atrium LA via a trans-septal approach (or through the atrial roof in a trans-atrial approach). In some embodiments, a distal portion of the guide catheter 312 can be positioned so that its distal-most end (e.g., the end furthest from the user) is in the left atrium LA. For example, the guide catheter 312 can extend to a location as shown in Figure 10A, or the guide catheter 312 can be positioned further in the left atrium LA to extend at least generally along the flow axis of the native cardiac valve (e.g., a generally vertical axis VA in Figure 10A). This alignment can be achieved via a combination of torqueing/steering the guide catheter 312 using the guide catheter handle 322, pre-shaping the end of the guide catheter 312, and/or flexing the guide catheter 312. However, in some embodiments, the guide catheter 312 may not have such complete steerability and its distal tip positioning may be more approximate such that the delivery catheter 314, the hub shaft 316, and/or the core shaft 318 (positioned within the guide catheter 312) can provide additional positioning at the desired location across the septum S.

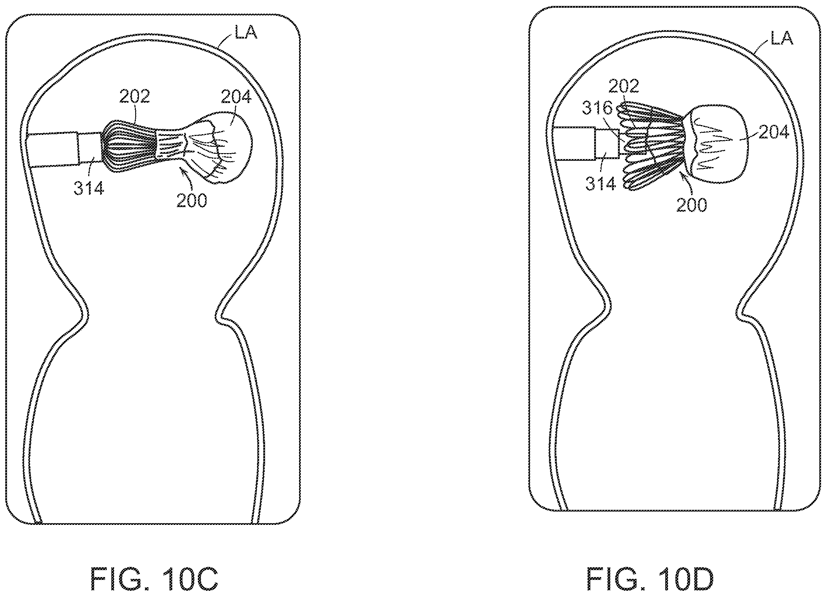

At block 942, the method 940 continues by advancing the delivery catheter 314 (including the implantable device 200 compressed therein), the hub shaft 316, and the core shaft 318 through the guide catheter 312 into the left atrium LA as shown in Figure 10B. Once positioned within the left atrium LA, the method 940 includes unsheathing the implantable device 200 from the delivery catheter 314 (block 943). For example, Figure 10C shows the implantable device 200 after being at least partially unsheathed, which allows the atrial-fixation member 202 and the baffle 204 to at least partially expand in the left atrium LA. The implantable device 200 can be unsheathed by proximally retracting the delivery catheter 314 relative to the implantable device 200. In other embodiments, the implantable device 200 can be unsheathed by distally advancing the implantable device 200 relative to the delivery catheter 314 (e.g., via advancement of the hub shaft 316 and the core shaft 318 relative to the delivery catheter 314) in addition to or alternatively to retracting the delivery catheter 314.

At block 944, the method 940 includes longitudinally/axially compressing (e.g., flattening, shortening) the implantable device 200. For example, Figure 10D shows the implantable device 200 after being compressed. Longitudinally compressing the implantable device 200 includes decreasing a distance between (i) the superior end portion S and the inferior end portion I and (ii) the plug assembly 438 and the core shaft 318. To longitudinally compress the implantable device 200, the user can move the hub shaft 316 and/or the core shaft 318 relative to one another to shorten the distance between the hub assembly 436 of the hub shaft 316 and the plug assembly 438 of the core shaft 318. For example, the user can move the hub shaft handle 326 and/or the core shaft handle 328 toward one another along the support assembly 320. In one aspect of the present technology, longitudinally compressing the implantable device 200 makes the implantable device 200 easier to rotate, translate, and/or position within the space constraints of the left atrium LA. In some embodiments, the implantable device 200 can be axially compressed by more than about 20%, more than about 50%, or more than about 70% relative to its length while compressed in the delivery catheter (block 942) and/or after being unsheathed in the left atrium LA (block 943). In other embodiments, block 943 can be omitted and the implantable device 200 need not be compressed longitudinally.

At block 945 the method 940 can include advancing the delivery catheter 314 distally toward the implantable device 200 such that the delivery catheter 314 is positioned near/over the hub assembly 436 which constrains the struts 206 at the superior end portion S of the implantable device 200. More specifically, the distal end portion of the delivery catheter 314 can be positioned over the hub assembly 436 while the compressed atrial fixation member 202 can at least partially surround the distal end portion of the delivery catheter 314. In some aspects of the present technology, positioning the delivery catheter 314 at least partially over the implantable device 200 can aid in steering the implantable device 200 within the left atrium LA by providing additional rigidity, pushability, and/or torqueability to the implantable device 200.

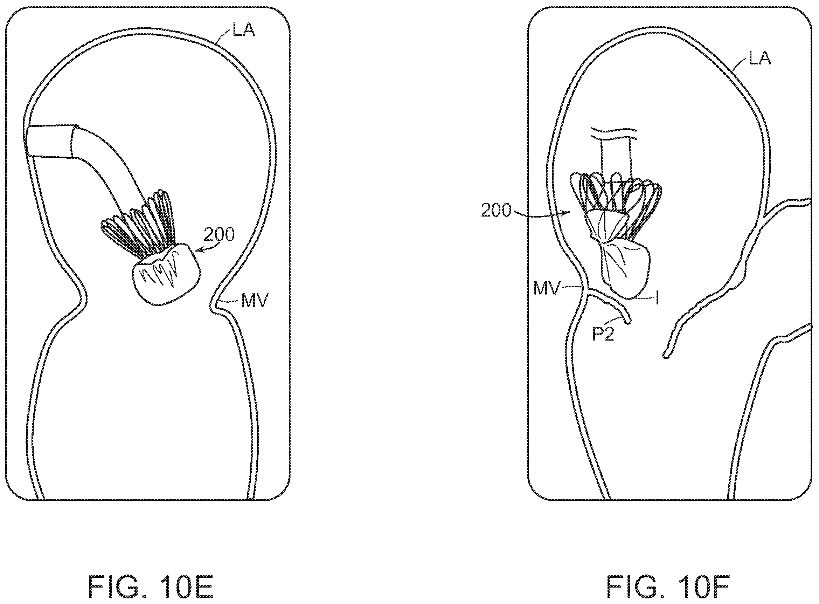

At block 946, the method 940 includes steering the implantable device 200 toward the mitral valve of the patient, such that the inferior end portion I of the implantable device 200 is directed toward the mitral valve annulus, and aligning the implantable device 200 with a portion of one or more desired native leaflets. For example, Figures 10E and 10F show the implantable device 200 during and after steering, respectively, the implantable device 200 toward a mitral valve MV and aligning the implantable device 200 with a native leaflet (e.g., the middle scallop P2 of the posterior leaflet) of the mitral valve MV. In some embodiments, the spine 378 of the delivery catheter 314 can help facilitate steering the implantable device 200 toward the mitral valve MV even within the relatively small space of the left atrium LA.

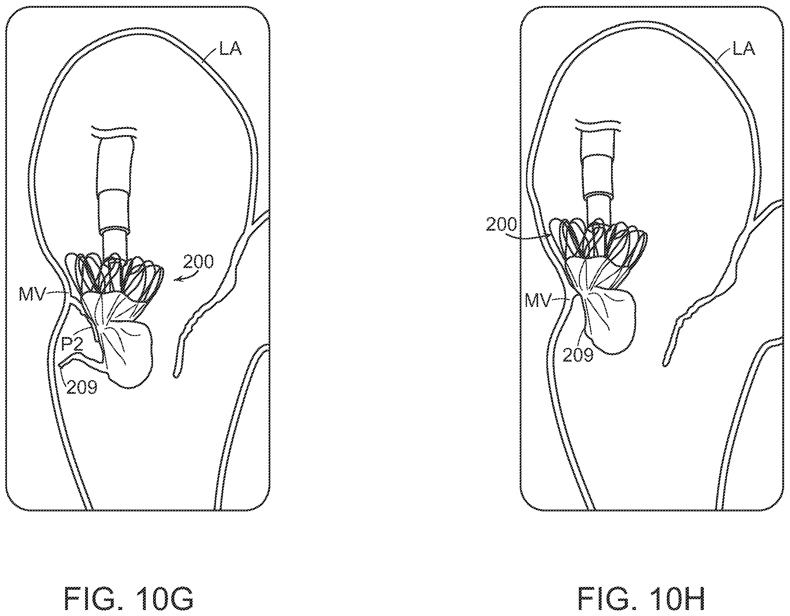

At block 947, after the implantable device 200 is rotationally and radially aligned with the portion of the desired leaflet, the method 940 includes advancing the implantable device 200 at least partially across the mitral valve MV and capturing the portion of the desired leaflet. For example, the implantable device 200 can be steered to the target site such that the implantable device 200 crosses the mitral valve annulus with the atrial-fixation member 202 positioned at least partially above the annulus in the left atrium LA and the coaptation member 204 positioned at or below the annulus in the left ventricle LV. Figures 10G and 10H show the implantable device 200 during and after capture of the middle scallop P2 of the posterior leaflet with the clip 209. In some embodiments, the clip 209 can be opened before or after crossing the mitral MV as shown in Figure 10G, and then closed to capture the middle scallop P2 of the posterior leaflet between the clip 209 and the baffle 204. As described in detail above with reference to Figures 2A, 2B, and 8A-8D, the user can open and close the clip 209 by actuating (e.g., depressing and then sliding) the clip actuator 806 of the core shaft handle 328. In some embodiments, the clip 209 can be opened to help orient the implantable device 200 within the left atrium LA.

In general, to steer the implantable device 200 to capture the desired native leaflet (blocks 946 and 947), the user can manipulate one or more of the handles 322-328. For example, the user can manipulate the guide catheter handle 322 and/or the delivery catheter handle 324 to advance and/or deflect the guide catheter 312 and/or the delivery catheter 314 in one or more directions-such as in a direction toward the mitral valve MV-to facilitate positioning of the implantable device 200. In some embodiments, re-advancing the delivery catheter 314 toward the implantable device 200, as shown in Figure 10E, facilitates positioning/steering of the implantable device via manipulation of the delivery catheter 314. Moreover, the implantable device 200 can be oriented as desired by rotating, for example, the hub shaft handle 326 and the core shaft handle 328 together relative to the delivery catheter 314. For example, with additional reference to Figure 3, the hub shaft handle 326 and the core shaft handle 328 can be rotated together about the second mount 327b and the third mount 327c, respectively. In some embodiments, because the hub shaft handle 326 and the core shaft handle 328 are coupled together via the rails 332, rotating one of the handles also rotates the other one of the handles.

Once the implantable device 200 is correctly positioned across the mitral valve MV with the coaptation member 204 in place with respect to the native leaflets, the method 940 can continue at block 948 by allowing the atrial-fixation member 202 to expand within the left atrium LA above the mitral valve MV by uncinching the atrial-fixation member 202. For example, Figure 10H shows the implantable device 200 after uncinching the atrial-fixation member 202. The user can uncinch the atrial-fixation member 202 by rotating the cinch actuator 808 to release tension from the cinch tendon 439. In some embodiments, the atrial-fixation member 202 does not contact the walls of the left atrium LA after being uncinched, or only a portion of the atrial-fixation member 202 contacts tissue within the left atrium LA (e.g., the posterior aspect of the left atrium LA).

At block 949, the method 940 includes determining/assessing whether the implantable device 200 is properly positioned and functioning properly. The performance of the implantable device 200, such as a reduction in an amount of mitral regurgitation, can be evaluated via transesophageal echocardiography ("TEE") imaging or another suitable technique.

At block 950, if the implantable device 200 is not functioning properly, the implantable device can be repositioned or recovered and removed from the patient. For example, the cinch actuator 808 can be actuated to radially compress the atrial-fixation member 202. Then, the clip 209 can be opened to release the native leaflet by actuating (e.g., sliding) the clip actuator 806 of the core shaft handle 328, and the implantable device 200 can be retracted into the left atrium LA. The implantable device 200 can then either be repositioned or removed. Prior to removal, in some embodiments the implantable device 200 can be elongated along its longitudinal axis by moving the hub shaft handle 326 and/or the core shaft handle 328 to move the hub shaft 316 and/or the core shaft 318 relative to one another to lengthen the distance between the hub assembly 436 of the hub shaft 316 and the plug assembly 438 of the core shaft 318. Elongating the implantable device 200 further radially compresses the implantable device 200. Finally, the implantable device 200 can be retracted into the guide catheter 312 and removed from the patient.

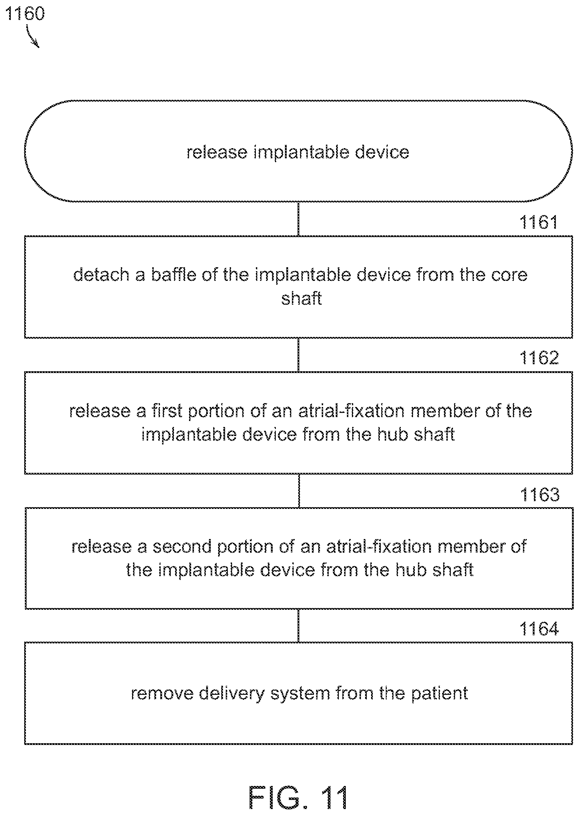

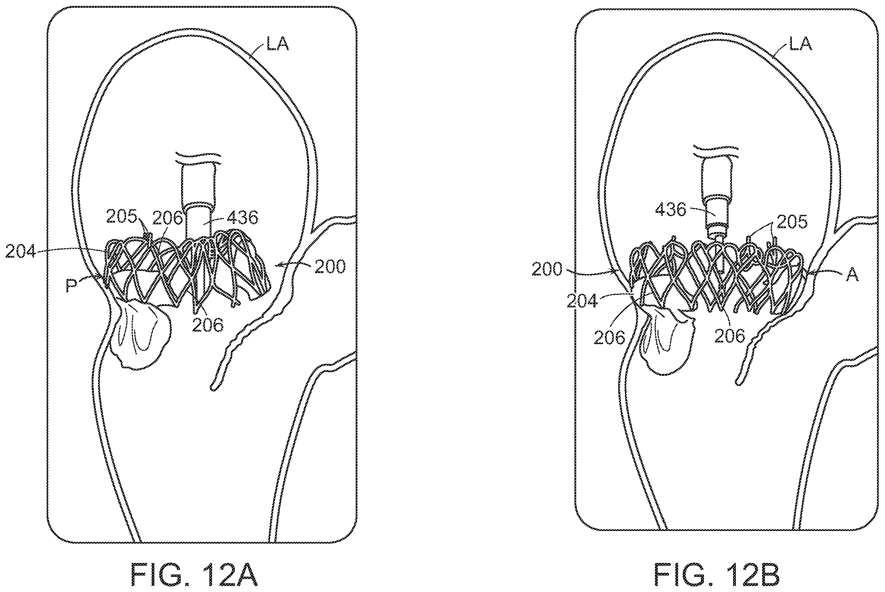

If the clinician decides that the implantable device 200 is functioning properly, the method 940 can continue to block 951 in which the implantable device 200 is fully released/detached from the delivery system 310. More specifically, Figure 11 is a flow diagram illustrating a process or method 1160 for releasing the implantable device at block 951 in accordance with embodiments of the present technology. Figures 12A-12D are side views illustrating the implantable device 200 and a distal portion of the delivery system 310 during various stages of the release method 1160 in accordance with embodiments of the present technology.

At block 1161, the release method 1160 includes detaching the baffle 204 from the delivery system 310, and more specifically from the core shaft 318. Detaching the baffle 204 can include actuating the baffle actuator 804 of the core shaft handle 328 to unscrew the baffle connection member 796 from the delivery attachment member 203 positioned within the hollow interior of the baffle 204. In some embodiments, the baffle 204 can be detached from the core shaft 318 prior to uncinching of the implantable device 200 (block 948 of Figure 9). In some embodiments, the tendons 439, 821 can be released (e.g., cut through the opening 805 in the core shaft handle 328) before detaching the baffle 204.

At block 1162, the release method 1160 includes releasing a first portion of the atrial-fixation member 202 from the hub assembly 436 of the hub shaft 316. For example, Figure 12A illustrates the implantable device 200 after releasing the connectors 205 positioned at the posterior side portion P of the implantable device 200. As described in detail above, the connectors 205 positioned at the posterior side portion P of the implantable device 200 can be released by actuating the ring gear 684 of the hub shaft handle 326 to drive the hub assembly 436 to the second position. With the first set of connectors 205 released, the first portion of the atrial-fixation member 202 (e.g., the struts 206 near the posterior side portion P) can further expand radially outwardly to contact and fixedly engage a wall of the left atrium LA.

At block 1163, the release method 1160 includes releasing a second portion of the atrial-fixation member 202 from the hub assembly 436 of the hub shaft 316. For example, Figure 12B illustrates the implantable device 200 after releasing the connectors 205 positioned at the anterior side portion A of the implantable device 200. As described in detail above, the connectors 205 positioned at the anterior side portion A of the implantable device 200 can be released by (i) pulling the release member 685 of the hub shaft handle 326 to disengage the release shaft 562 from the lock pin 558 and then (ii) actuating the ring gear 684 of the hub shaft handle 326 to drive the hub assembly 436 to the third position. After release, the second portion of the atrial-fixation member 202 (e.g., the struts 206 near the anterior side portion A) can contact and fixedly engage the wall of the left atrium LA. In some embodiments, the position and/or orientation of the implantable device 200 can be manipulated after releasing the first portion of the atrial-fixation member 202 and before releasing the second portion of the atrial-fixation member 202.

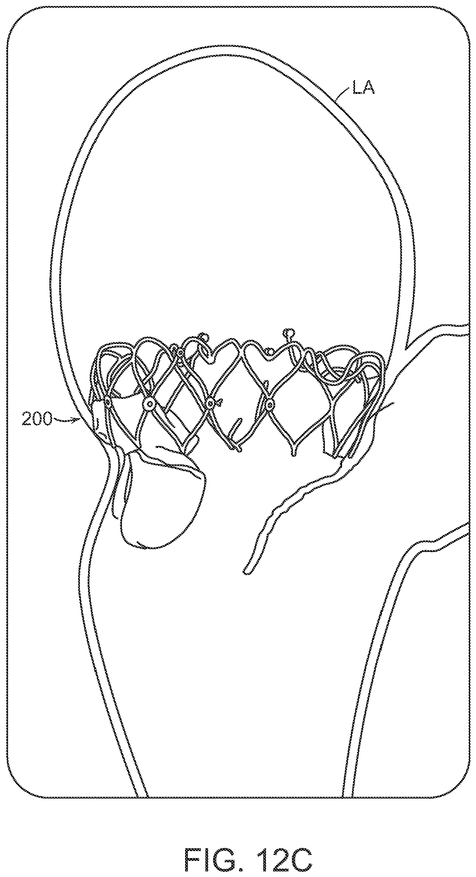

At block 1164, the release method 1160 includes removing the delivery system 310 from the patient. For example, the catheters 312-314 can be individually or collectively withdrawn from the patient such that only the implantable device 200 remains, as shown in Figure 12C.

Referring to Figures 2A-12C together, the delivery system 310 has several advantages that enable the delivery system 310 to reliably implant the implantable device 200 at the mitral valve MV to have a proper position and orientation. For example, the delivery system 310 allows for the separate control of the atrial-fixation member 202 and the coaptation member 204, and allows these components to be separately released from the delivery system 310. Additionally, the delivery system 310 facilitates the longitudinal compression of the implantable device 200 that allows the implantable device 200 to be navigated through the tight anatomy of the left atrium LA. Moreover, the hub assembly 436 allows the atrial-fixation member 202 to be released in two stages to facilitate the proper engagement of the atrial-fixation member 202 with the wall of the left atrium LA. Other advantages are described in detail throughout.

### IV. Selected Additional Embodiments of Hub Assemblies

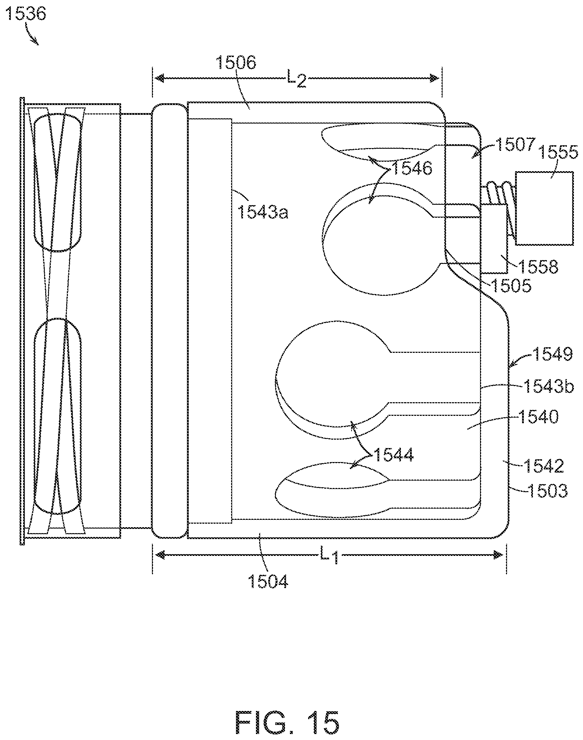

Figure 15 is an enlarged side view of a hub assembly 1536 configured in accordance with additional embodiments of the present technology. The hub assembly 1536 can (i) include features several features generally similar or identical to the features of the hub assembly 436 described in detail above with reference to Figures 4-5F, (ii) operate generally similarly or identically to the hub assembly 436, and/or (iii) can be integrated into the system 310 in a generally similar or identical manner. For example, with additional reference to Figures 2A and 2B, the hub assembly 1536 is configured to similarly receive and secure the connectors 205 of the implantable device 200 and to provide for a controlled, two-stage release of the atrial-fixation member 202 in which the struts 206 at the posterior side portion P are released from the hub assembly 1536 and allowed to expand before the struts 206 at the anterior side portion A (e.g., as shown in detail in Figures 12A and 12B). For ease, the hub assembly 1536 is described herein with respect to the features of the implantable device 200 of Figures 2A and 2B, though the hub assembly 1536 can be used to retain and/or deploy other implantable devices.

More particularly, the hub assembly 1536 can include an inner hub component 1540 and an outer hub component 1542 including a distal opening 1549. The outer hub component 1542 is shown as partially transparent in Figure 15 for clarity. The inner hub component 1540 can include (i) a proximal side or edge 1543a, (ii) a distal side or edge 1543b, (iii) first recesses 1544 extending proximally from the distal edge 1543b and configured to receive corresponding ones of the connectors 205 positioned at the anterior side portion A of the implantable device 200 (Figures 2A and 2B), and (iv) second recesses 1546 extending proximally from the distal edge 1543b and configured to receive corresponding ones of the connectors 205 positioned at the posterior side portion P of the implantable device 200. In Figure 15, the hub assembly 1536 is arranged in a first position in which the outer hub component 1542 is positioned to extend over each of the first and second recesses 1544, 1546 such that the first and second recesses 1544, 1556 are positioned proximal of the distal opening 1549. Thus, when the hub assembly 1536 is in the first position, the connectors 205 of the implantable device 200 are secured/restrained in the first and second recesses 1544, 1546 by the outer hub component 1542.

The hub assembly 1536 can further include a lead screw 1555 and/or other drive member configured to translate the inner hub component 1540 relative to the outer hub component 1542, thereby moving the hub assembly 1536 from the first position to a second position and from the second position to a third position (e.g., via actuation of the hub shaft handle 316 shown in Figures 6A-6C). As described in detail above with reference to Figures 4-5F and 12A-12C, in the second position, at least a portion of each of the second recesses 1546 can be positioned sufficiently distal of the distal opening 1549 of the outer hub component 1542 that the connectors 205 positioned at the posterior side portion P of the implantable device 200 are no longer constrained by the outer hub component 1542, and thus free to disengage from the hub assembly 1536 to partially deploy the implantable device 200. In the third position, at least a portion of each of the first recesses 1544 can be positioned sufficiently distal of the distal opening 1549 that the connectors 205 positioned at the anterior side portion A of the implantable device 200 are no longer constrained by the outer hub component 1542, and thus free to disengage the hub assembly 1536 to further deploy the implantable device 200. In some embodiments, the hub assembly 1536 can further include a lock pin 1558 configured to halt further advancement of the inner hub component 1542 from the second position to the third position.

In the illustrated embodiment, the outer hub component 1542 has a distal edge 1503 including a stepped portion 1505 defining a cut-out region 1507. The outer hub component 1542 can have a generally cylindrical shape apart from the cut-out region 1507. The stepped portion 1505 and the cut-out region 1507 can divide the outer hub component 1542 into a first portion 1504 positioned and/or aligned over the first recesses 1544 and a second portion 1506 positioned and/or aligned over the second recesses 1546. The first portion 1504 can have a side length L₁ that is longer than a side length L₂ of the second portion 1506. When the inner hub component 1540 is translated distally relative to the outer hub component 1542 (e.g., to the second position), the shorter side length L₂ provided by the cut-out region 1507 allows the second recesses 1546 (and the connectors 205 positioned therein) to extend out of the distal opening 1549 and become uncovered by the outer hub component 1542 before the first recesses 1544 (and the connectors 205 positioned therein). Accordingly, the differential lengths L₁ and L₂ of the outer hub component 1542 can facilitate the staged release of the atrial-fixation member 202 while traversing a shorter overall axial length than a similar hub assembly with a uniform length. In some aspects of the present technology, the shorter axial length can help improve steerability of the hub assembly 1636 in tight anatomies and/or help facilitate quicker deployment of the implantable device 200, and/or (iii) enable

In the illustrated embodiment, the second recesses 1546 have a length (e.g., extending proximally from the distal edge 1543b toward the proximal edge 1543a) that is shorter than a length of the first recesses 1544. As described in detail above with reference to Figures 5A-6C and 12A-12C, this arrangement can further facilitate the two-stage release of the atrial-fixation member 202. In some embodiments, the stepped portion 1505 allows a difference between the lengths of the first and second recesses 1544, 1546 to be reduced as compared to a difference between the lengths of the first and second recesses 544, 546 shown in Figures 5A-5C, while still providing the same deployment profile as the hub assembly 536 (e.g., having the same timing and actuation mechanics at the hub shaft handle 326 shown in Figures 6A-6C). In some embodiments, the first and second recesses 1544, 1546 can have the same or generally similar sizes and dimensions (e.g., lengths) such that the two-stage release of the atrial-fixation member 202 is facilitated only or substantially by the differential lengths L₁ and L₂ of the outer hub portion 1542.

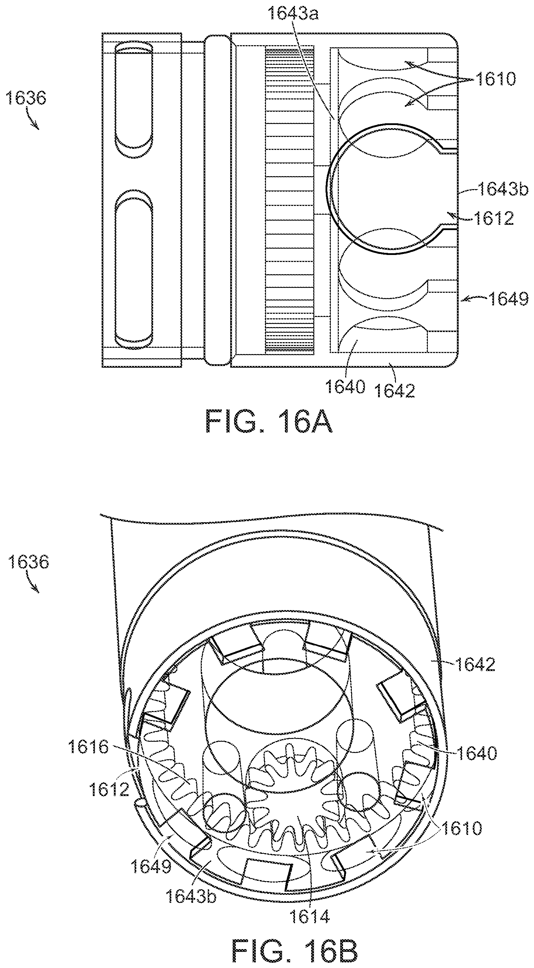

Figures 16A and 16B are an enlarged side view and a front isometric view, respectively, of a hub assembly 1636 in accordance with additional embodiments of the present technology. The hub assembly 1636 can (i) include features several features generally similar or identical to the hub assembly 436 and/or the hub assembly 1536 described in detail above with reference to Figures 4-5F and 15, (ii) operate generally similarly or identically to the hub assembly 436 and/or the hub assembly 1536, and/or (iii) can be integrated into the system 310 in a generally similar or identical manner. For example, with additional reference to Figures 2A and 2B, the hub assembly 1636 is configured to similarly receive and secure the connectors 205 of the implantable device 200 and to provide for a controlled, multi-stage release of the atrial-fixation member 202 in which the struts 206 at the posterior side portion P are released from the hub assembly 1536 and allowed to expand before the struts 206 at the anterior side portion A (e.g., as shown in detail in Figures 12A and 12B). For ease, the hub assembly 1636 is described herein with respect to the features of the implantable device 200 of Figures 2A and 2B, though the hub assembly 1636 can be used to retain and/or deploy other implantable devices.

More particularly, referring to Figures 16A and 16B together, the hub assembly 1636 can include an inner hub component 1640 and an outer hub component 1642 including a distal opening 1649. The outer hub component 1642 is shown as partially transparent in Figure 16A, and the inner hub component 1640 is shown as partially transparent in Figure 16B for clarity. The inner hub component 1640 can include (i) a proximal side or edge 1643a, (ii) a distal side or edge 1643b, (iii) and a plurality of recesses 1610 extending proximally from the distal edge 1643b and configured to receive corresponding ones of the connectors 205. In some embodiments, each of the recesses 1610 can have the same configuration (e.g., size, shape, dimensions). The outer hub component 1642 can include an opening 1612 (e.g., an aperture, a slot) that is configured (e.g., sized, shaped, dimensioned) to be positioned over a corresponding one of the recesses 1610 to allow one of the connectors 205 secured therein to release from within the recess 1610 and disengage the hub assembly 1636.

Referring to Figure 16B, the outer hub component 1642 is rotatably mounted relative to the inner hub component 1640. For example, in the illustrated embodiment the hub assembly 1636 further includes a pinion gear 1614, and the outer hub component 1642 includes a ring gear 1616 that is operably coupled to the pinion gear 1614. In some embodiments, the pinion gear 1614 is operably coupled to the drive shaft 552 (Figures 5D-5F), which extends from the hub assembly 1636, through the hub shaft 316 (Figure 4), and to the hub shaft handle 326 (Figures 6A-6C). Referring to Figures 6A-6C and 16B together, the hub shaft handle 326 can be manipulated/actuated by a user to actuate the drive shaft 552 to drive (e.g., rotate) the pinion gear 1614 to rotate the outer hub component 1642 relative to the inner hub component 1640 to release the connectors 205 of the implantable device 200 (Figures 2A and 2B) during device deployment.

More specifically, referring to Figures 2A, 2B, 16A, and 16B together, the outer hub component 1642 can be rotated to align the opening 1612 over a corresponding one of the recesses 1610 to release the connector 205 positioned therein. In operation, the hub assembly 1636 can initially be positioned as shown in Figure 16A with the opening 1612 misaligned with the recesses 1610 such that each of the connectors 205 is constrained by the outer hub component 1642 in a corresponding one of the recesses 1610. The outer hub component 1642 can then be rotated to sequentially align the opening 1612 over individual ones of the recesses 1610 including the connectors 205 at the posterior side portion P to sequentially release those ones of the connectors 205, before being further rotated to sequentially align the opening 1612 over individual ones of the recesses 1610 including the connectors 205 at the anterior side portion A to sequentially release those ones of the connectors 205. In this manner, the hub assembly 1636 can provide for the sequential, multi-stage release of the connectors at the posterior side portion P and the anterior side portion A of the implantable device 200.

In the illustrated embodiment, the opening 1612 is configured (e.g., shaped and sized) to be sequentially positioned over individual ones of the recesses 1610 to release the connectors 205 positioned therein. In some aspects of the present technology, the individual (e.g., one-by-one) release of the connectors 205 can improve control of the deployment of the atrial-fixation member 202. In other embodiments, the opening 1612 can be sized to be positioned over two or more of the recesses 1610 at the same time to, for example, facilitate the simultaneous release of multiple ones of the connectors 205 secured therein. In some embodiments, the outer hub component 1642 has two or more openings 1612 spaced about its circumference such that relative rotation of the inner and outer hub components 1640, 1642 provides for simultaneous release of multiple ones of the connectors 205 secured therebetween.

In some embodiments, the hub assembly 1636 can have a relatively shorter length than the hub assemblies 436 and 1536 described in detail above with reference to Figures 5A-5F and 15, as the outer hub component 1642 need not be translated relative to the inner hub component 1640 to release the connectors 205. In some aspects of the present technology, reducing the reduced length of the hub assembly 1636 can help improve steerability of the hub assembly 1636 in tight anatomies.

### VI. Conclusion

The above detailed description of embodiments of the technology are not intended to be exhaustive or to limit the technology to the precise form disclosed above. Although specific embodiments of, and examples for, the technology are described above for illustrative purposes, various equivalent modifications are possible within the scope of the technology as those skilled in the relevant art will recognize. For example, although steps are presented in a given order, alternative embodiments can perform steps in a different order. The various embodiments described herein can also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms can also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications can be made without deviating from the technology. Further, while advantages associated with some embodiments of the technology have been described in the context of those embodiments, other embodiments can also exhibit such advantages, and not all embodiments need necessarily exhibit such advantages to fall within the scope of the technology. Accordingly, the disclosure and associated technology can encompass other embodiments not expressly shown or described herein.

## Claims

1. A delivery system (310) for endovascularly implanting a valve repair device (200) at a mitral valve, the delivery system (310) comprising:
a delivery catheter (314) having a distal portion configured to be endovascularly delivered to a left atrium, wherein the distal portion is configured to hold the valve repair device (200) in a delivery state;
a hub shaft (316) extending through the delivery catheter (314), the hub shaft (316) having a proximal portion and a distal portion;
a hub assembly (436) coupled to the distal portion of the hub shaft (316), wherein-
the hub assembly (436) includes an inner hub component (540) that is movable relative to an outer hub component (542) between a first position, a second position, and a third position,
in the first position, the hub is configured to secure a first end portion of the valve repair device (200) between the inner and outer hub components (540, 542),
movement of the inner hub component (540) from the first position to the second position is configured to release a first side portion of the first end portion of the valve repair device (200), and
movement of the inner hub component (540) from the second position to the third position is configured to release a second side portion of the first end portion of the valve repair device (200) to release the first end portion from the hub; and
a core shaft (318) extending through and independently movable with respect to the hub shaft (316), the core shaft (318) having a plug assembly (438) configured to releasably engage a second end portion of the valve repair device (200).

2. The delivery system (310) of claim 1 wherein the inner hub component (540) includes a plurality of recesses (544, 546) configured to receive corresponding ones of a plurality of connectors (205) of the valve repair device (200).

3. The delivery system (310) of claim 2 wherein the recesses include a group of first recesses (546) and a group of second recesses (544), wherein the second recesses (544) each have a different shape than the first recesses (546).

4. The delivery system (310) of claim 3 wherein the inner hub component (540) includes a distal edge (543b) and a proximal edge (543a), wherein the first recesses (546) extend from the distal edge (543b) partially toward the proximal edge (543a), and wherein the second recesses (544) extend from the distal edge (543b) toward the proximal edge (543a) farther than the first recesses (546).

5. The delivery system (310) of claim 3 wherein-
in the first position, the first and second recesses (546, 544) are covered by the outer hub component (542);
in the second position, (a) the first recesses (546) are positioned distal of the outer hub component (542) to permit the first side portion of the valve repair device (200) to release and (b) the second recesses (544) are covered by the outer hub component (542); and
in the third position, the first and second recesses (546, 544) are positioned distal of the outer hub component (542) to permit the second side portion of the valve repair device (200) to release.

6. The delivery system (310) of claim 3 wherein the group of first recesses (546) is circumferentially spaced apart from the group of second recesses (544) about the inner hub component (540).

7. The delivery system (310) of claim 1 wherein the hub assembly (436) further comprises a lock pin (558) configured to engage the inner and outer hub components (540, 542) in the second position to inhibit movement of the inner hub component (540) from the second position to the third position.

8. The delivery system (310) of claim 7, further comprising a release shaft (562) operably coupled to the lock pin (558), wherein the release shaft (562) is actuatable to permit the lock pin (558) to disengage the outer hub component (542) to permit movement of the inner hub component (540) from the second position to the third position.

9. The delivery system (310) of claim 8 wherein the lock pin (558) includes a release lumen (563) configured to receive the release shaft (562) therein, wherein the release shaft (562) is configured to bias the lock pin (558) radially outward to engage the outer hub component (542), and wherein the release shaft (562) is removable from the release lumen (563) to permit the lock pin (558) to disengage the outer hub component (542).

10. The delivery system (310) of claim 1, further comprising a drive shaft (552), wherein the hub assembly (436) includes a lead screw (555) operably coupling the drive shaft (552) to the inner hub component (540), and wherein rotation of the drive shaft (552) rotates the lead screw (555) to move the inner hub component (540) between the first, second, and/or third positions.

11. The delivery system (310) of claim 10 wherein the inner hub component (540) includes a stop surface, and wherein the lead screw (555) includes a stop portion configured to engage the stop surface in the third position to inhibit further movement of the inner hub component (540) from the third position in a direction away from the second position.

12. The delivery system (310) of claim 1 wherein:
the hub shaft (316) and the core shaft (318) are independently movable relative to one another to change an axial length of the valve repair device (200).

13. The delivery system (310) of claim 1 wherein the first end portion of the valve repair device (200) comprises an atrial-fixation member (202), and wherein the second end portion of the valve repair device (200) comprises a coaptation member (204) extending from the atrial-fixation member (202).

14. The delivery system (310) of claim 1, further comprising:
a hub shaft handle (326) coupled to a proximal portion of the hub shaft (316); and
a drive shaft (552) extending through the hub shaft (316) and operably coupling the hub assembly (436) to the hub shaft handle (326), wherein the hub shaft handle (326) includes a drive actuator configured to drive the drive shaft (552) to move the hub assembly (436) between the first, second, and/or third positions.

15. The delivery system (310) of claim 14 wherein the drive actuator includes a ring gear (684).

## Patentansprüche

1. Abgabesystem (310) zum endovaskulären Implantieren einer Klappenreparaturvorrichtung (200) an einer Mitralklappe, wobei das Abgabesystem (310) Folgendes umfasst:
einen Abgabekatheter (314) mit einem distalen Abschnitt, der konfiguriert ist, um endovaskulär in einen linken Vorhof abgegeben zu werden, wobei der distale Abschnitt konfiguriert ist, um die Klappenreparaturvorrichtung (200) in einem Abgabezustand zu halten;
einen Nabenschaft (316), der sich durch den Abgabekatheter (314) hindurch erstreckt, wobei der Nabenschaft (316) einen proximalen Abschnitt und einen distalen Abschnitt aufweist;
eine Nabenanordnung (436), die mit dem distalen Abschnitt des Nabenschafts (316) gekoppelt ist, wobei-
die Nabenanordnung (436) eine innere Nabenkomponente (540) einschließt, die relativ zu einer äußeren Nabenkomponente (542) zwischen einer ersten Position, einer zweiten Position und einer dritten Position beweglich ist,
die Nabe in der ersten Position konfiguriert ist, um einen ersten Endabschnitt der Klappenreparaturvorrichtung (200) zwischen der inneren und der äußeren Nabenkomponente (540, 542) zu sichern,
die Bewegung der inneren Nabenkomponente (540) von der ersten Position in die zweite Position konfiguriert ist, um einen ersten Seitenabschnitt des ersten Endabschnitts der Klappenreparaturvorrichtung (200) freizugeben, und
die Bewegung der inneren Nabenkomponente (540) von der zweiten Position in die dritte Position konfiguriert ist, um einen zweiten Seitenabschnitt des ersten Endabschnitts der Klappenreparaturvorrichtung (200) freizugeben, um den ersten Endabschnitt von der Nabe freizugeben; und
einen Kernschaft (318), der sich durch den Nabenschaft (316) hindurch erstreckt und in Bezug auf diesen unabhängig beweglich ist, wobei der Kernschaft (318) eine Stopfenanordnung (438) aufweist, die konfiguriert ist, um einen zweiten Endabschnitt der Klappenreparaturvorrichtung (200) lösbar in Eingriff zu bringen.

2. Abgabesystem (310) nach Anspruch 1, wobei die innere Nabenkomponente (540) eine Vielzahl von Aussparungen (544, 546) einschließt, die konfiguriert sind, um entsprechende einer Vielzahl von Konnektoren (205) der Klappenreparaturvorrichtung (200) aufzunehmen.

3. Abgabesystem (310) nach Anspruch 2, wobei die Aussparungen eine Gruppe von ersten Aussparungen (546) und eine Gruppe von zweiten Aussparungen (544) einschließen, wobei die zweiten Aussparungen (544) jeweils eine andere Form aufweisen als die ersten Aussparungen (546).

4. Abgabesystem (310) nach Anspruch 3, wobei die innere Nabenkomponente (540) eine distale Kante (543b) und eine proximale Kante (543a) einschließt, wobei sich die ersten Aussparungen (546) von der distalen Kante (543b) teilweise zu der proximalen Kante (543a) hin erstrecken, und wobei sich die zweiten Aussparungen (544) von der distalen Kante (543b) weiter zu der proximalen Kante (543a) hin erstrecken als die ersten Aussparungen (546).

5. Abgabesystem (310) nach Anspruch 3, wobei-
die ersten und zweiten Aussparungen (546, 544) in der ersten Position durch die äußere Nabenkomponente (542) bedeckt sind;
(a) die ersten Aussparungen (546) in der zweiten Position distal von der äußeren Nabenkomponente (542) positioniert sind, um dem ersten Seitenabschnitt der Klappenreparaturvorrichtung (200) zu ermöglichen, freizugeben, und (b) die zweiten Aussparungen (544) von der äußeren Nabenkomponente (542) bedeckt sind; und
die ersten und die zweiten Aussparungen (546, 544) in der dritten Position distal von der äußeren Nabenkomponente (542) positioniert sind, um dem zweiten Seitenabschnitt der Klappenreparaturvorrichtung (200) zu ermöglichen, freizugeben.

6. Abgabesystem (310) nach Anspruch 3, wobei die Gruppe von ersten Aussparungen (546) in Umfangsrichtung von der Gruppe von zweiten Aussparungen (544) um die innere Nabenkomponente (540) herum beabstandet ist.

7. Abgabesystem (310) nach Anspruch 1, wobei die Nabenanordnung (436) ferner einen Verriegelungsstift (558) umfasst, der konfiguriert ist, um in der zweiten Position die innere und die äußere Nabenkomponente (540, 542) in Eingriff zu bringen, um eine Bewegung der inneren Nabenkomponente (540) aus der zweiten Position in die dritte Position zu verhindern.

8. Abgabesystem (310) nach Anspruch 7, ferner umfassend einen Freigabeschaft (562), der mit dem Verriegelungsstift (558) funktionsfähig gekoppelt ist, wobei der Freigabeschaft (562) betätigbar ist, um dem Verriegelungsstift (558) zu ermöglichen, die äußere Nabenkomponente (542) außer Eingriff zu bringen, um eine Bewegung der inneren Nabenkomponente (540) von der zweiten Position in die dritte Position zu ermöglichen.

9. Abgabesystem (310) nach Anspruch 8, wobei der Verriegelungsstift (558) ein Freigabelumen (563) einschließt, das konfiguriert ist, um den Freigabeschaft (562) darin aufzunehmen, wobei der Freigabeschaft (562) konfiguriert ist, um den Verriegelungsstift (558) radial nach außen vorzuspannen, um die äußere Nabenkomponente (542) in Eingriff zu bringen, und wobei der Freigabeschaft (562) aus dem Freigabelumen (563) entfernbar ist, um dem Verriegelungsstift (558) zu ermöglichen, die äußere Nabenkomponente (542) außer Eingriff zu bringen.

10. Abgabesystem (310) nach Anspruch 1, ferner umfassend einen Antriebsschaft (552), wobei die Nabenanordnung (436) eine Führungsschraube (555) einschließt, die den Antriebsschaft (552) mit der inneren Nabenkomponente (540) funktionsfähig koppelt, und wobei eine Drehung des Antriebsschafts (552) die Führungsschraube (555) dreht, um die innere Nabenkomponente (540) zwischen der ersten, zweiten und/oder dritten Position zu bewegen.

11. Abgabesystem (310) nach Anspruch 10, wobei die innere Nabenkomponente (540) eine Anschlagoberfläche einschließt und wobei die Führungsschraube (555) einen Anschlagabschnitt einschließt, der konfiguriert ist, um die Anschlagoberfläche in der dritten Position in Eingriff zu bringen, um eine weitere Bewegung der inneren Nabenkomponente (540) aus der dritten Position in einer Richtung weg von der zweiten Position zu verhindern.

12. Abgabesystem (310) nach Anspruch 1, wobei:
der Nabenschaft (316) und der Kernschaft (318) unabhängig voneinander relativ zueinander beweglich sind, um die axiale Länge der Klappenreparaturvorrichtung (200) zu verändern.

13. Abgabesystem (310) nach Anspruch 1, wobei der erste Endabschnitt der Klappenreparaturvorrichtung (200) ein Vorhoffixierungselement (202) umfasst und wobei der zweite Endabschnitt der Klappenreparaturvorrichtung (200) ein Koaptationselement (204) umfasst, das sich von dem Vorhoffixierungselement (202) erstreckt.

14. Abgabesystem (310) nach Anspruch 1, ferner umfassend:
ein Nabenschaftgriff (326), der mit einem proximalen Abschnitt des Nabenschafts (316) gekoppelt ist; und
einen Antriebsschaft (552), der sich durch den Nabenschaft (316) hindurch erstreckt und die Nabenanordnung (436) mit dem Nabenschaftgriff (326) funktionsfähig koppelt, wobei der Nabenschaftgriff (326) einen Antriebsaktuator einschließt, der konfiguriert ist, um den Antriebsschaft (552) anzutreiben, um die Nabenanordnung (436) zwischen der ersten, zweiten und/oder dritten Position zu bewegen.

15. Abgabesystem (310) nach Anspruch 14, wobei der Antriebsaktuator ein Ringzahnrad (684) einschließt.

## Revendications

1. Système d'administration (310) destiné à implanter par voie endovasculaire un dispositif de réparation de valve (200) au niveau d'une valve mitrale, le système d'administration (310) comprenant :
un cathéter d'administration (314) ayant une partie distale conçue pour être administrée par voie endovasculaire à une oreillette gauche, dans lequel la partie distale est conçue pour maintenir le dispositif de réparation de valve (200) dans un état d'administration ;
un arbre de moyeu (316) s'étendant à travers le cathéter d'administration (314), l'arbre de moyeu (316) ayant une partie proximale et une partie distale ;
un ensemble moyeu (436) accouplé à la partie distale de l'arbre de moyeu (316), dans lequel-
l'ensemble moyeu (436) comporte un composant de moyeu interne (540) qui est mobile par rapport à un composant de moyeu externe (542) entre une première position, une deuxième position et une troisième position,
dans la première position, le moyeu est conçu pour fixer une première partie d'extrémité du dispositif de réparation de valve (200) entre les composants de moyeu interne et externe (540, 542),
un mouvement du composant de moyeu interne (540) de la première position à la deuxième position est conçu pour libérer une première partie latérale de la première partie d'extrémité du dispositif de réparation de valve (200), et
un mouvement du composant de moyeu interne (540) de la deuxième position à la troisième position est conçu pour libérer une seconde partie latérale de la première partie d'extrémité du dispositif de réparation de valve (200) afin de libérer la première partie d'extrémité du moyeu ; et
un arbre central (318) s'étendant à travers l'arbre de moyeu (316) et pouvant se déplacer indépendamment par rapport à celui-ci, l'arbre central (318) ayant un ensemble bouchon (438) conçu pour venir en prise de manière amovible avec une seconde partie d'extrémité du dispositif de réparation de valve (200).

2. Système d'administration (310) selon la revendication 1, dans lequel le composant de moyeu interne (540) comporte une pluralité d'évidements (544, 546) conçus pour recevoir des connecteurs correspondants d'une pluralité de connecteurs (205) du dispositif de réparation de valve (200).

3. Système d'administration (310) selon la revendication 2, dans lequel les évidements comportent un groupe de premiers évidements (546) et un groupe de seconds évidements (544), dans lequel les seconds évidements (544) ont chacun une forme différente des premiers évidements (546).

4. Système d'administration (310) selon la revendication 3, dans lequel le composant de moyeu interne (540) comporte un bord distal (543b) et un bord proximal (543a), dans lequel les premiers évidements (546) s'étendent du bord distal (543b) partiellement vers le bord proximal (543a), et dans lequel les seconds évidements (544) s'étendent du bord distal (543b) vers le bord proximal (543a) plus loin que les premiers évidements (546).

5. Système d'administration (310) selon la revendication 3, dans lequel-
dans la première position, les premiers et les seconds évidements (546, 544) sont recouverts par le composant de moyeu externe (542) ;
dans la deuxième position, (a) les premiers évidements (546) sont positionnés de manière distale par rapport au composant de moyeu externe (542) pour permettre à la première partie latérale du dispositif de réparation de valve (200) de se libérer et (b) les seconds évidements (544) sont recouverts par le composant de moyeu externe (542) ; et
dans la troisième position, les premiers et les seconds évidements (546, 544) sont positionnés de manière distale par rapport au composant de moyeu externe (542) pour permettre à la seconde partie latérale du dispositif de réparation de valve (200) de se libérer.

6. Système d'administration (310) selon la revendication 3, dans lequel le groupe de premiers évidements (546) est espacé circonférentiellement du groupe de seconds évidements (544) autour du composant de moyeu interne (540).

7. Système d'administration (310) selon la revendication 1, dans lequel l'ensemble moyeu (436) comprend en outre une goupille de verrouillage (558) conçue pour venir en prise avec les composants de moyeu interne et externe (540, 542) dans la deuxième position afin d'empêcher le mouvement du composant de moyeu interne (540) de la deuxième position à la troisième position.

8. Système d'administration (310) selon la revendication 7, comprenant en outre une tige de libération (562) accouplée de manière opérationnelle à la goupille de verrouillage (558), dans lequel la tige de libération (562) est actionnable pour permettre à la goupille de verrouillage (558) de se désolidariser du composant de moyeu externe (542) afin de permettre le mouvement du composant de moyeu interne (540) de la deuxième position à la troisième position.

9. Système d'administration (310) selon la revendication 8, dans lequel la goupille de verrouillage (558) comporte une lumière de libération (563) conçue pour recevoir la tige de libération (562) à l'intérieur de celle-ci, dans lequel la tige de libération (562) est conçue pour solliciter la goupille de verrouillage (558) radialement vers l'extérieur pour venir en prise avec le composant de moyeu externe (542), et dans lequel la tige de libération (562) est amovible de la lumière de libération (563) pour permettre à la goupille de verrouillage (558) de se désolidariser du composant de moyeu externe (542).

10. Système d'administration (310) selon la revendication 1, comprenant en outre un arbre d'entraînement (552), dans lequel l'ensemble moyeu (436) comporte une vis-mère (555) accouplant de manière opérationnelle l'arbre d'entraînement (552) au composant de moyeu interne (540), et dans lequel la rotation de l'arbre d'entraînement (552) met en rotation la vis-mère (555) pour déplacer le composant de moyeu interne (540) entre la premier, la deuxième, et/ou la troisième position.

11. Système d'administration (310) selon la revendication 10, dans lequel le composant de moyeu interne (540) comporte une surface d'arrêt, et dans lequel la vis-mère (555) comporte une partie d'arrêt conçue pour venir en prise avec la surface d'arrêt dans la troisième position afin d'empêcher tout mouvement supplémentaire du composant de moyeu interne (540) de la troisième position dans une direction s'éloignant de la deuxième position.

12. Système d'administration (310) selon la revendication 1, dans lequel :
l'arbre de moyeu (316) et l'arbre central (318) sont indépendamment mobiles l'un par rapport à l'autre pour modifier une longueur axiale du dispositif de réparation de valve (200).

13. Système d'administration (310) selon la revendication 1, dans lequel la première partie d'extrémité du dispositif de réparation de valve (200) comprend un élément de fixation auriculaire (202), et dans lequel la seconde partie d'extrémité du dispositif de réparation de valve (200) comprend un élément de coaptation (204) s'étendant à partir de l'élément de fixation auriculaire (202).

14. Système d'administration (310) selon la revendication 1, comprenant en outre :
une poignée d'arbre de moyeu (326) accouplée à une partie proximale de l'arbre de moyeu (316) ; et
un arbre d'entraînement (552) s'étendant à travers l'arbre de moyeu (316) et accouplant de manière opérationnelle l'ensemble moyeu (436) à la poignée d'arbre de moyeu (326), dans lequel la poignée d'arbre de moyeu (326) comporte un actionneur d'entraînement conçu pour entraîner l'arbre d'entraînement (552) afin de déplacer l'ensemble moyeu (436) entre la première, la deuxième et/ou la troisième position.

15. Système d'administration (310) selon la revendication 14, dans lequel l'actionneur d'entraînement comporte une couronne dentée (684).
